# NOUVEAU FASCICULE DE BREVET EUROPEEN

(11) **EP 0 815 835 B3**
(45) Date de publication du présent fascicule: **19.08.2009**
(45) Mention de la délivrance du brevet: 04.11.1998
(21) Numéro de dépôt: 97401328.6
(22) Date de dépôt: 12.06.1997
(51) Int. Cl.: A61K 7/42

(54) **Compositions comprenant du 4-tert-butyl-4'-methoxydibenzoylmethane, un dérivé de 1,3,5-triazine et un (alpha-cyano)-beta, beta'-diphénylacrylate d'alkyle et utilisations**
Zusammensetzungen enthaltend einen 4-ter-Butyl-4'-Methoxydibenzoylmethan, einen 1,3,5-Triazinderivat und einen (Alpha-cyano)-beta, beta'-Diphenylacrylsäure-Alkylester sowie ihre Verwendung
Compositions containing a 4-tertiar-butyl-4'-methoxydibenzoylmethan, a 1,3,5-triazine derivative and an alkyl(alpha-cyano)-beta, beta'-diphenylacrylate and uses thereof

(30) Priorité: 01.07.1996 FR 9608172
(43) Date de publication de la demande: 07.01.1998
(73) Titulaire: L'ORÉAL, 75008 Paris (FR)
(72) Inventeur: Allard, Delphine, 92700 Colombes (FR); Forestier, Serge, 77410 Claye-Souilly (FR)
(74) Mandataire: Miszputen, Laurent

(56) Documents cités:
- EP-A- 0 685 221
- EP-A- 0 689 828

## Description

La présente invention concerne de nouvelles compositions cosmétiques et/ou dermatologiques (ci-après appelées compositions antisolaires) destinées à la protection de la peau et/ou des cheveux contre les rayonnements UV, en particulier le rayonnement solaire. Plus précisément, elle concerne de nouvelles compositions cosmétiques et/ou dermatologiques présentant une photostabilité améliorée et comprenant, dans un support cosmétiquement et/ou dermatologiquement acceptable, l'association de trois filtres particuliers.

L'invention concerne également l'utilisation de ces compositions dans le domaine cosmétique et/ou dermatologique.

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Il est donc souhaitable de filtrer aussi le rayonnement UV-A.

Ainsi, dans le but d'assurer une protection de la peau et des cheveux contre l'ensemble du rayonnement UV qui soit la plus complète et la plus efficace possible, on utilise généralement dans la fabrication des compositions antisolaires des associations de filtres actifs dans l'UVA et de filtres actifs dans l'UVB.

A cet égard, le 4-tert-butyl-4'-méthoxydibenzoylméthane, vendu sous la dénomination commerciale « Parsol 1789 » par la société Givaudan, est un filtre actif dans l'UVA particulièrement intéressant compte tenu de son fort pouvoir d'absorption intrinsèque.

De même, les dérivés de 1,3,5-triazine, et en particulier la 2,4,6-tris[p-(2'-éthylhexyl-1'-oxycarbonyl)anilino]-1,3,5-triazine, vendue sous la dénomination commerciale « Uvinul T 150 » par la société BASF, possèdent un fort pouvoir absorbant des UVB et il serait donc très intéressant de pouvoir les utiliser, en association avec le 4-tert-butyl-4'-méthoxydibenzoylméthane cité ci-dessus dans le but d'obtenir des produits offrant une protection large et efficace dans l'ensemble du rayonnement UV.

Il est connu du document EP-A-0 685 221 des compositions cosmétiques comprenant de la 2,4,6-tris[p-(2'-éthylhexyl-1'-oxycarbonyl)anilino]-1,3,5-triazine et de l'α-cyano-β,β'-diphénylacrylate de 2-éthylhexyle. Il est également connu du document EP-A-0 689 828 des compositions cosmétiques comprenant de la 2,4,6-tris[p-(2'-éthylhexyl-1'-oxycarbonyl)anilino]-1,3,5-triazine et du malate de dioctyle.

Toutefois, la Demanderesse a constaté que ces dérivés de 1,3,5-triazine, lorsqu'ils sont en présence de 4-tert-butyl-4'-méthoxydibenzoylméthane et sous irradiation UV, présentent l'inconvénient de se dégrader chimiquement de façon importante. Dans ces conditions, l'association des deux filtres ne permet plus une protection solaire large prolongée de la peau et des cheveux.

Or, à la suite d'importantes recherches menées dans le domaine de la photoprotection évoqué ci-dessus, la Demanderesse a maintenant découvert que l'introduction d'un β,β'-diphénylacrylate d'alkyle ou α-cyano-β,β'-diphénylacrylate d'alkyle dans une composition contenant du 4-tert-butyl-4'-méthoxydibenzoylméthane en association avec au moins un dérivé de 1,3,5-triazine, et en particulier avec la 2,4,6-tris[p-(2'-éthylhexyl-1'-oxycarbonyl)anilino]-1,3,5-triazine, permettait d'améliorer de façon tout à fait remarquable la stabilité de ce dérivé de 1,3,5-triazine au sein de telles compositions, et donc l'efficacité globale de ces compositions.

Cette découverte est à la base de l'invention.

La présente invention a donc pour objet de nouvelles compositions cosmétiques et/ou dermatologiques comprenant, dans un support cosmétiquement et/ou dermatologiquement acceptable, i) du 4-tert-butyl-4'-méthoxydibenzoylméthane, ii) au moins un dérivé de 1,3,5-triazine répondant à la formule (I) suivante : 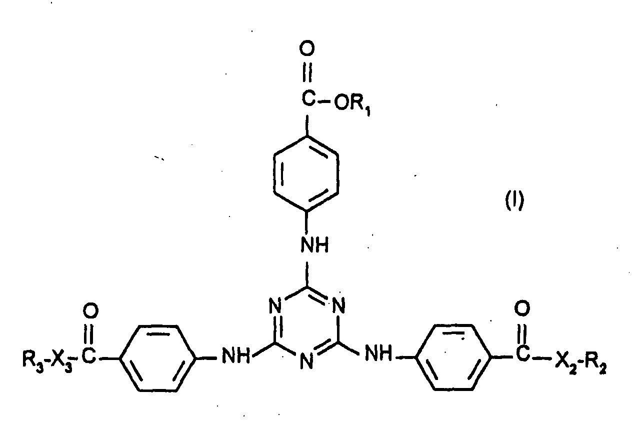 dans laquelle :
- X₂ et X₃, identiques ou différents, représentent l'oxygène ou le radical -NH-;
- R₁, R₂ et R₃, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé; un radical de formule (II), (III) ou (IV) suivantes : 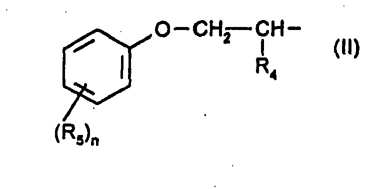 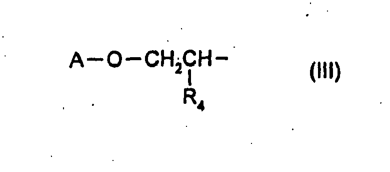 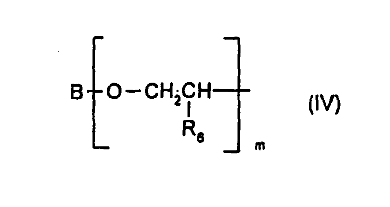
dans lesquelles :
- R₄ est l'hydrogène ou un radical méthyle;
- R₅ est un radical alkyle en C₁-C₉;
- n est un nombre entier allant de 0 à 3;
- m est un nombre entier allant de 1 à 10;
- A est un radical alkyle en C₄-C₈ ou un radical cycloalkyle en C₅-C₈;
- B est choisi parmi: un radical alkyle linéaire ou ramifié en C₁-C₈; un radical cycloalkyle en C₅-C₈; un radical aryle éventuellement substitué par un ou plusieurs radicaux alkyles en C₁-C₄;
- R₈ est l'hydrogène ou un radical méthyle,
   et iii) au moins un β,β'-diphénylacrylate d'alkyle ou α-cyano-β,β'-diphénylacrylate d'alkyle de formule (V) suivante : 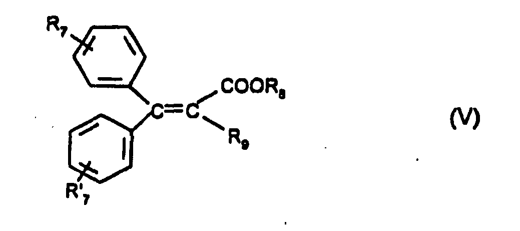
dans laquelle:
- R₇ et R'₇, identiques ou différents, sont en position méta ou para et sont choisis parmi : l'hydrogène; un radical alcoxy en C₁-C₈ à chaîne droite ou ramifiée; un radical alkyle en C₁-C₄ à chaîne droite ou ramifiée;
- R₈ représente un radical alkyle en C₁-C₁₂ à chaîne droite ou ramifiée;
- R₉ représente un atome d'hydrogène ou un radical -CN, lesdites compositions étant exemptes de p-méthoxycinnamate de 2-éthylhexyle, à l'exception de ce qui est exclus dans la revendication

Ainsi, selon la présente invention, on peut réaliser des compositions cosmétiques et/ou dermatologiques contenant du 4-tert-butyl-4'-méthoxydibenzoylméthane en association avec au moins un dérivé de 1,3,5-triazine, compositions
dans lesquelles la concentration en dérivé de 1,3,5-triazine reste relativement constante même si ces compositions sont soumises à l'action de la lumière.

Par ailleurs, les β,β'-diphénylacrylates d'alkyle ou α-cyano-β,β'diphénylacrylates d'alkyle utilisés dans le cadre de la présente invention présentent l'avantage de posséder un bon pouvoir filtrant intrinsèque qui contribue à la protection contre les UV conférée par les compositions, et, de plus, l'ensemble du système filtrant [4-tert-butyl-4'-méthoxydienzoylméthane + dérivé de 1,3,5-triazine + (β,β'-diphénylacrylate ou α-cyano-β,β'-diphénylacrylate d'alkyle)] s'avère présenter globalement une très bonne stabilité sous l'action des UV (photostabilité), ce qui constitue un autre avantage supplémentaire des compositions selon l'invention.

La présente invention a encore pour objet l'utilisation d'un β,β'-diphénylacxylate d'alkyle ou α-cyano-β,β'-diphénylacrylate d'alkyle dans, ou pour la fabrication de, compositions cosmétiques et/ou dermatologiques contenant du 4-tert-butyl-4'-méthoxydibenzoylméthane en association avec au moins un dérivé de 1,3,5-triazine tel que défini ci-dessus en vue d'améliorer dans lesdites compositions la stabilité au rayonnement UV (photostabilité) dudit dérivé de 1,3.5-triazine.

La présente invention a également pour objet un procédé pour améliorer la stabilité au rayonnement UV (photostabilité), et donc l'efficacité, d'une composition cosmétique et/ou dermatologique comprenant du 4-tert-butyl-4'-méthoxydbenzoytméthane et un dérivé de 1,3,5-triazine tel que défini ci-dessus, en particulier la 2,4,6-tris[p-(2'-éthylhexyl-1'-oxycarbonyl)anilino]-1,3,5-triazine, ledit procédé consistant à introduire dans ladite composition une quantité efficace d'un β,β'-diphénylacrylate d'alkyle ou α-cyano-β,β'-diphénylacrylate d'alkyle.

D'autres caractéristiques, aspects et avantages de la présente invention apparaîtront à la lecture de la description détaillée qui va suivre.

Un premier composé des compositions selon l'invention est donc le 4-tert-butyl-4'-méthoxydibenzoylméthene. C'est un filtre bien connu en soi qui possède un fort pouvoir absorbant dans les UVA avec un maximum à 358 nm. Il est proposé à la vente sous la dénomination commerciale de "PARSOL 1789" par la Société Givaudan et répond à la formule développée (VI) suivante :

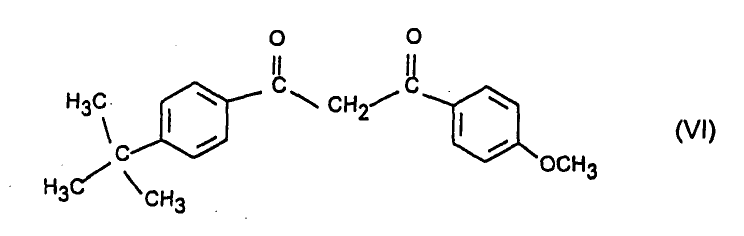

Le 4-tert-butyl-4'-méthoxydibenzoylméthane peut être présent dans les compositions de l'invention à une teneur allant de 0,2 % à 15 % en poids, par rapport au poids total de la composition. De préférence, cette teneur va de 0,5 % à 10 %.

Un deuxième composé des compositions visées par la présente invention est un dérivé particulier de 1,3,5-triazine. Ainsi, les dérivés de 1,3,5-triazine utilisables dans le cadre de la présente invention sont choisis parmi ceux répondant à la formule (I) suivante : 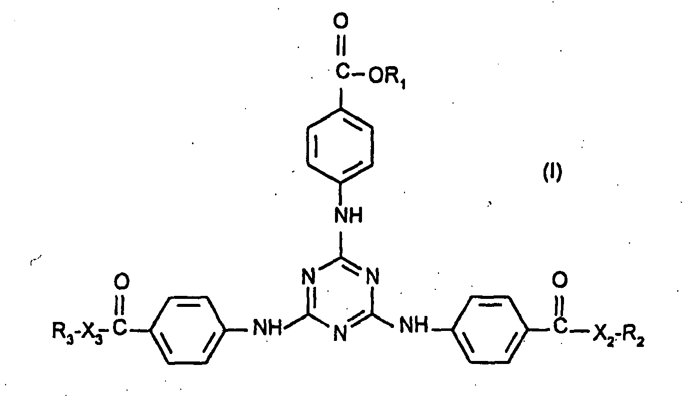 dans laquelle:
- X₂ et X₃, identiques ou différents, représentent l'oxygène ou le radical -NH-;
- R₁, R₂ et R₃, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé; un radical de formule (II), (III) ou (IV) suivantes : 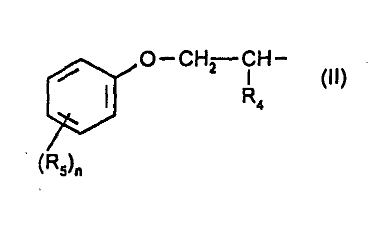 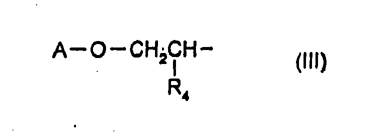 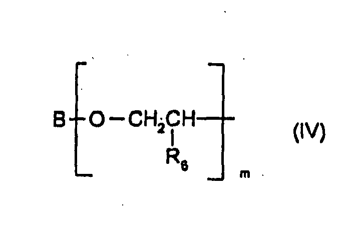
dans lesquelles :
- R₄ est l'hydrogène ou un radical méthyle;
- R₅ est un radical alkyle en C₁-C₉;
- n est un nombre entier allant de 0 à 3;
- m est un nombre entier allant de 1 à 10;
- A est un radical alkyle en C₄-C₈ ou un radical cycloalkyle en C₅-C₈;
- B est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄;
- R₆ est l'hydrogène ou un radical méthyle.

Une première famille plus particulièrement préférée de dérivés de 1,3,5-triazine est celle, notamment décrite dans le document EP-A-0 517 104, des 1,3,5-triazines répondant à la formule (I) ci-dessus et présentant l'ensemble des caractéristiques suivantes :
- X₂ et X₃ sont identiques et représentent l'oxygène;
- R₁ est choisi parmi : un radical cycloalkyle en C₅-C₁₂ éventuellement substitué par un ou plusieurs radicaux alkyles en C₁-C₄; un radical de formule (II), (III) ou (IV) ci-dessus dans lesquelles :

- B est un radical alkyle en C₁-C₄;
- R₆ est le radical méthyle;
- R₂ et R₃, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (II), (III) ou (IV) ci-dessus dans lesquelles :

- B est un radical alkyle en C₁-C₄;
- R₆ est le radical méthyle.

Une deuxième famille préférée de dérivés de 1,3,5-triazine selon l'invention est celle, notamment décrite dans le document EP-A-0 570 838, des 1,3,5-triazines répondant à la formule (I) et présentant l'ensemble des caractéristiques suivantes :
- X₃ est le radical -NH-;
- R₃ est choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄;
- R₁ est choisi parmi : l'hydrogène; un métal alcalin; un radical ammonium; un radical de formule (IV); un radical alkyle linéaire ou ramifié en C₁-C₁₈; un radical cycloalkyle en C₅-C₁₂ éventuellement substitué avec un ou plusieurs radicaux alkyles en C₁-C₄;
- si X₂ est le radical -NH-, alors R₂ est choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄;
- si X₂ est l'oxygène, alors R₂ est choisi parmi l'hydrogène; un métal alcalin; un radical ammonium; un radical de formule (IV); un radical alkyle linéaire ou ramifié en C₁-C₁₈; un radical cycloalkyle en C₅-C₁₂ éventuellement substitué avec un ou plusieurs radicaux alkyles en C₁-C₄.

Une 1,3,5-triazine particulièrement préférée de cette deuxième famille est celle répondant à la formule suivante : 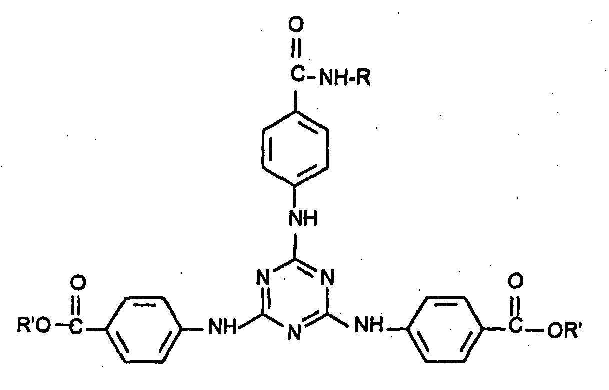 dans laquelle R' désigne un radical éthyl-2 hexyle et R désigne un radical tert. butyle.

Une troisième famille préférée de composés est celle, notamment décrite dans le document US 4,724,137, des 1,3,5-triazines répondant à la formule (I) et présentant l'ensemble des caractéristiques suivantes :
- X₂ et X₃ sont identiques et représentent l'oxygène;
- R₁, R₂ et R₃ sont identiques et représentent un radical alkyle en C₆-C₁₂ ou un radical polyoxyéthyléné comprenant de 1 à 6 unités d'oxyde d'éthylène et dont le groupe OH terminal est méthylé.

Une 1,3,5-triazine particulièrement préférée de cette troisième famille est la 2,4,6-tris[p-(2'-éthylhexyl-1'-oxycarbonyl)anilino]-1,3,5-triazine qui est un filtre connu en soi, actif dans l'UV-B, se présentant sous une forme solide, et qui est vendu notamment sous la dénomination commerciale de "UVINUL T 150" par la Société BASF Ce produit répond à la formule suivante : 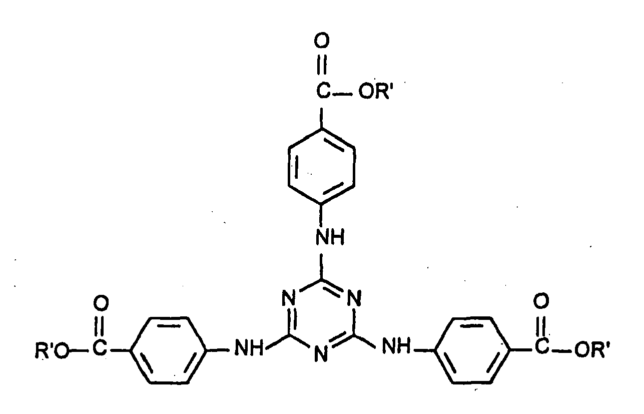 dans laquelle R' désigne un radical 2-éthyl hexyle.

Le ou les dérivés de 1,3,5-triazine sont généralement présents dans les compositions de l'invention à une teneur pouvant aller de 0,5 % à 20 %, de préférence de 1 % à 10 % en poids, par rapport au poids total de la composition.

Un troisième composé absolument essentiel des compositions selon l'invention est un composé de la famille comprenant les β,β'-diphénylacrylates d'alkyle et les α-cyano-β,β'-diphénylacrylates d'alkyle. Les β,β'-diphénylacrylates d'alkyle et α-cyano-β,β'-diphénylacrylates d'alkyle utilisables selon la présente invention sont choisis parmi ceux répondant à la formule (V) suivante : 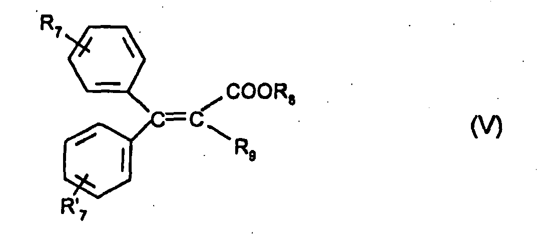 dans laquelle :
- R₇ et R'₇, identiques ou différents, sont en position méta ou para et sont choisis parmi : l'hydrogène; un radical alcoxy en C₁-C₈ à chaîne droite ou ramifiée; un radical alkyle en C₁-C₄ à chaîne droite ou ramifiée;
- R₈ représente un radical alkyle en C₁-C₁₂ à chaîne droite ou ramifiée;
- R₉ représente un atome d'hydrogène ou un radical -CN.

Parmi les β,β'-diphénylacrylates d'alkyle et α-cyano-β,β'-diphénylacrylates d'alkyle utilisables selon la présente invention, on préfère plus particulièrement l'α-cyano-β,β' diphénylacrylate de 2-éthylhexyle ou encore l'α-cyano-β, β'-diphénylacrylate d'éthyle.

L'α-cyano-β,β' diphénylacrylate de 2-éthylhexyle, encore appelé octocrylène, est connu pour être un filtre lipophile absorbant dans les UVB. Il est disponible commercialement et vendu notamment sous la dénomination de "UVINUL N 539" par la Société BASF. Il répond à la formule suivante : 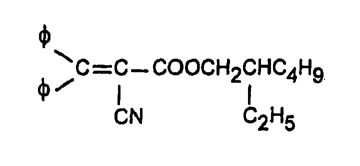 dans laquelle φ désigne un radical phényle.

L'α-cyano-β,β' diphénylacrylate d'éthyle, encore appelé étocrylène, est également un filtre liposoluble absorbant dans les UVB. Il est disponible commercialement et vendu notamment sous la dénomination de "UVINUL N 35" par la Société BASF Il répond à la formule suivante : 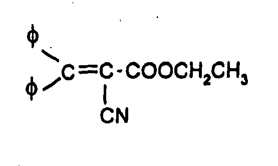 dans laquelle φ désigne un radical phényle.

Ainsi, lorsqu'on ajoute en quantité suffisante un β,β'-diphénylacrylate d'alkyle ou α-cyano-β,β'-diphénylacrylate d'alkyle à une composition antisolaire contenant du 4-tert-butyl-4'-méfhoxydibenzoylméthane et un dérivé de 1,3,5-triazine tel que défini ci-dessus, on observe une augmentation de la stabilité dudit dérivé de 1,3,5-triazine à la lumière, et donc une amélioration de l'efficacité de la composition antisolaire au cours du temps.

De préférence, le β,β'-diphénylacrylate d'alkyle ou α-cyano-β,β'-diphénylacrylate d'alkyle est présent dans les compositions selon l'invention à une teneur au moins égale à 0,5 % en poids, par rapport au poids total de la composition. De préférence encore, cette teneur va de 0,5 % à 20 % en poids, par rapport au poids total de la composition.

Les compositions cosmétiques et/ou dermatologiques visées par la présente invention peuvent bien entendu contenir un ou plusieurs filtres solaires complémentaires actifs dans l'UVA et/ou l'UVB (absorbeurs), hydrophiles ou lipophiles, autres bien sûr que les trois filtres mentionnés ci-avant. Ces filtres complémentaires peuvent être notamment choisis parmi les dérivés cinnamiques, les dérivés salicyliques, les dérivés du benzylidène camphre, les dérivés de benzimidazole, les dérivés de triazine autres que ceux ci-avant mentionnés, les dérivés de la benzophénone, les dérivés de β,β'-diphénylacrylate autres que ceux ci-avant mentionnés, les dérivés de l'acide p-aminobenzoîque, les polymères filtres et silicones filtres décrits dans la demande WO-93/04665. D'autres exemples de filtres organiques sont donnés dans la demande de brevet EP-A 0 487 404.

De préférence, les compositions selon l'invention ne comprennent pas de p-méthoxycinnamate de 2-éthylhexyle. En effet, le p-méthoxycinnamate de 2-éthylhexyle peut déstabiliser des compositions comprenant du 4-tert-butyl-4'-méthoxydibenzoylméthane et un dérivé de 1,3,5-triazine telles que les compositions conformes à l'invention.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques et/ou dermatologiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflexion et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A-0 518 772 et EP-A- 0 518 773.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline); végétales (huile d'amande douce, de macadamia, de pépin de cassis, de jojoba) ; synthétiques comme le perhydrosqualène, les alcools, les acides ou les esters gras (comme le benzoate d'alcools en C12-C15 vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir le ou les éventuels composés complémentaires cités ci-dessus (en particulier les filtres complémentaires) et/ou leurs quantités de manière telle que les propriétés avantageuses attachées intrinsèquement à l'association ternaire conforme à l'invention ne soient pas, ou substantiellement pas, altérées par la ou les adjonctions envisagées.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique et/ou dermatologique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloratian des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95 % en poids, de préférence de 70 à 90 % en poids, par rapport à l'ensemble de la formulation, la phase huileuse (comprenant notamment les filtres lipophiles) de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10% en poids, par rapport à l'ensemble de la formulation.

La présente invention porte encore sur un procédé de traitement cosmétique pour protéger la peau et/ou les cheveux contre le rayonnement UV, en particulier le rayonnement solaire, **caractérisé en ce qu**'il consiste à appliquer sur ces derniers une quantité efficace d'une composition cosmétique telle que définie ci-dessus.

Un exemple concret, mais nullement limitatif, illustrant l'invention, va maintenant être donné.

### EXEMPLE:

On a réalisé quatre émulsions huile-dans-eau A, B , C et D dont le support commun présente la composition suivante (les quantités sont exprimées en % de poids par rapport au poids total de la composition) :

| | |
|---|---|
| mélange d'alcool cétylstéarylique et d'alcool cétylstéarylique oxyéthyléné (33 OE) 80/20 vendu sous la dénomination commerciale « DEHSCONET 390 » par Tensia | 7 % |
| - mélange de mono et distéarate de glycérol vendu sous la dénomination commerciale « CERASYNTH SD » par ISP | 2 % |
| - alcool cétylique | 1,5 % |
| - polydiméthylsiloxane vendu sous la dénomination commerciale « DC 200 Fluid » par Dow Corning | 1,5 % |
| - benzoate d'alcools C12/C15 vendu sous la dénomination commerciale « FINSOLV TN » par Finetex | 15 % |
| - acide éthylène diamine tétracétique, sel disodique, 2 H₂0 | 0,1 % |
| - glycérine | 20 % |
| - conservateurs qs | |
| - eau déminéralisée qsp | 100 % |

L'émulsion A (comparative) comprend en outre un dérivé de 1,3,5 triazine qui est la 2,4,6-tris[p-(2'-éthylhexyl-1'-oxycarbonyl)anilino]-1,3,5-triazine (Uvinul T 150). L'émulsion B, également comparative, contient de l'Uvinul T 150 en association avec du 4-tert-butyl-4'-méthoxydibenzoylméthane (Parsol 1789). L'émulsion C, selon l'invention, comprend, outre de l'Uvinul T 150 et du Parsol 1789, de l'α-cyano-β,β-diphénylacrylate de 2-éthylhexyle (Uvinul N 539). L'émulsion D, comparative, comprend quant à elle de l'Uvinul T 150 en association avec le Parsol 1789 mais avec un filtre UVB classique qui est l'octylméthoxycinnamate (Parsol MCX).

Les compositions des émulsions A, B, C et D au niveau des différents filtres cités ci-dessus qu'elles contiennent sont rassemblées dans le tableau (I) ci-dessous (les quantités sont exprimées en % de poids par rapport au poids total de la composition) :

**Tableau (I)**

| Filtre | Emulsion A (comparative) | Emulsion B (comparative) | Emulsion C (invention) | Emulsion D (comparative) |
|---|---|---|---|---|
| Uvinul T 150 | 1,5 % | 1,5 % | 1,5 % | 1,5% |
| Parsol 1789 | - | 0,5 % | 0,5 % | 0,5 % |
| Uvinul N 539 | - | - | 10% | - |
| Parsol MCX | - | - | - | 10 % |

Pour chacune de ces émulsions, on a déterminé le pourcentage de 2,4,6-tris[p-(2'-éthylhexyl-1'-oxycarbonyl)anilino]-1,3,5-triazine résiduelle après irradiation par des UV selon le protocole suivant : pour chaque formule, on a préparé quatre échantillons témoins et quatre échantillons tests. On a déposé sur des plaques de PMMA (polyméthacrylate de méthyle) dépolies, préalablement rincées à l'eau puis séchées, 16 mg de formule qu'on a étalée sur une surface de 2 cm x 4 cm.

Puis on a irradié les plaques (SUNTEST CPS Heraeus) pendant 4 heures dans une enceinte dont la température est régulée aux environs de 35-40 °C afin de simuler une irradiation UV naturelle en conservant les plaques témoins à l'obscurité pendant le temps d'irradiation des autres plaques.

On a ensuite dosé les échantillons de la manière suivante : on a procédé à l'extraction des filtres en immergeant chaque plaque dans 55 ml d'isopropanol afin de solubiliser les filtres. Les plaques et le solvant contenant les filtres ont ensuite été traités aux ultrasons pendant 5 minutes pour assurer une extraction efficace. Les solutions obtenues sont analysées par chromatographie en phase liquide haute performance.

Pour chaque formule testée, le taux de 2,4,6-tris[p-(2'-éthylhexyl-1'-oxycarbonyl)anilino]-1,3,5-triazine résiduelle après irradiation est donné par le rapport de sa concentration dans l'échantillon irradié à sa concentration dans l'échantillon non irradié.

Les résultats en pourcentage de 2,4,6-tris[p-(2'-éthylhexyl-1'-oxycarbonyl)anilino]-1,3,5-triazine restante sont consignés dans le tableau (II) suivant :

**Tableau (II)**

| Emulsion | Uvinul T 150 résiduel |
|---|---|
| Emulsion A (comparative) | 80 % |
| Emulsion B (comparative) | 68 % |
| Emulsion C (invention) | 99 % |
| Emulsion D (comparative) | 79 % |

## Revendications (Revendications pour l'(les) Etat(s) contractant(s) suivant(s): ES, GB, FR, IT)

1. Composition cosmétique et/ou dermatologique comprenant, dans un support cosmétiquement et/ou dermatologiquement acceptable, i) du 4-tert-butyl-4'-méthoxydibenzoyl-méthane, ii) au moins un dérivé de 1,3,5-triazine répondant à la formule (I) suivante : 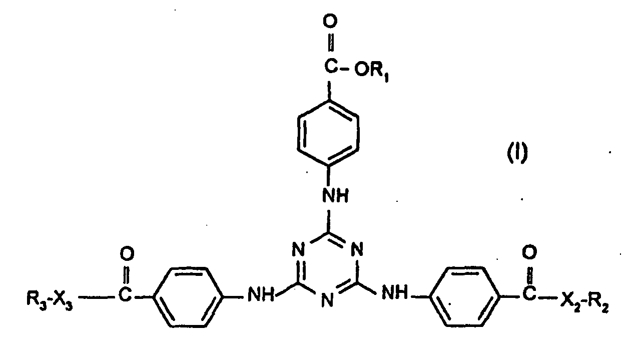 dans laquelle :
- X₂ et X₃, identiques ou différents, représentent l'oxygène ou le radical -NH-;
- R₁, R₂ et R₃, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé; un radical de formule (II), (III) ou (IV) suivantes : 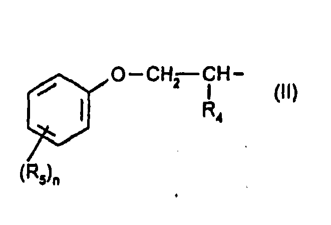 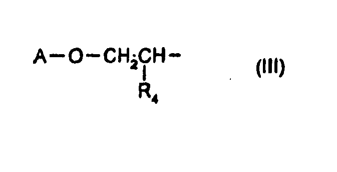 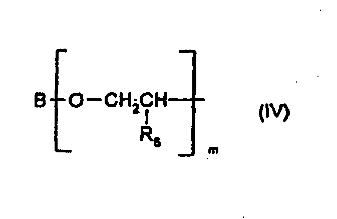
dans lesquelles:
- R₄ est l'hydrogène ou un radical méthyle;
- R₅ est un radical alkyle en C₁-C₉;
- n est un nombre entier allant de 0 à 3;
- m est un nombre entier allant de 1 à 10;
- A est un radical alkyle en C₄-C₈ ou un radical cycloalkyle en C₅-C_{8;}
- B est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄;
- R₆ est l'hydrogène ou un radical méthyle,
et iii) au moins un β.β'-diphénylacrylate d'alkyle ou α-cyano-β,β'-diphénylacrylate d'alkyle de formule (V) suivante : 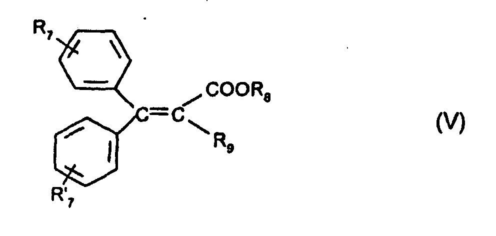
dans laquelle :
- R₇ et R'₇, identiques ou différents, sont en position méta ou para et sont choisis parmi : l'hydrogène; un radical alcoxy en C₁-C₈ à chaîne droite ou ramifiée; un radical alkyle en C₁-C₄ à chaîne droite ou ramifiée;
- R₈ représente un radical alkyle en C₁-C₁₂ à chaîne droite ou ramifiée;
- R₉ représente un atome d'hydrogène ou un radical -CN,
ladite composition étant exempte de p-méthoxycinnamate de 2-éthylhexyle **et sous réserve que la composition soit différente de la formulation suivante dans laquelle les quantités des différents ingrédients sont indiquées en pourcentage en poids par rapport au poids total de la composition**
| **Ingrédients** | **Quantités** |
|---|---|
| **Composant I, filtres UV** | |
| 4-Methylbenzylidene Camphor (Eusolex 6300) - Filtre UV-B | 3,0 |
| 2,4,6-tris[p-(2'-éthylhexyl-1'-oxycarbonyl)anilino]-1,3,5-triazine (Uvinul T150) - Filtre UV-B | 1,5 |
| 2-Ethylhexyl Salicylate (Neo Heliopan OS) - Filtre UV-B | 4,0 |
| α-cyano-β,β' diphénylacrylate de 2-éthylhexyle (Neo Heliopan 303) Filtre UV-B | 4,0 |
| 4-tert-butyl-4'-méthoxydibenzoylméthane (Parsol 1789) - Filtre UV-A | 2,0 |
| **Composant II, composant gras** | |
|---|---|
| C₁₂-C₁₅ Alkyl Benzoate (Finsolv TN) | 12,0 |
| **Composant III** | |
|---|---|
| Huile parfum | 0,4 |
| Conservateur | q.s. |
| **Composant IV** | |
|---|---|
| Carbomer | 0,8 |
| Glycerine | 3,0 |
| EDTA | 0,1 |
| Eau | qsp 100 |
| **Composant V** | |
|---|---|
| Eau | 20,0 |
| Acide novantisolique (filtre UVB hydrosoluble) | 2,0 |
| Agent neutralisant (notamment NaOH, Triethanolamine | q.s |

2. Composition selon la revendication 1, **caractérisée par le fait que** le dérivé de 1,3,5-triazine est choisi parmi ceux présentant l'ensemble des caractéristiques suivantes :
- X₂ et X₃ sont identiques et représentent l'oxygène;
- R₁ est choisi parmi : un radical cycloalkyle en C₅-C₁₂ éventuellement substitué par un ou plusieurs radicaux alkyles en C₁-C₄; un radical de formule (II), (III) ou (IV) dans lesquelles:
- B est un radical alkyle en C₁-C₄;
- R₆ est le radical méthyle;
- R₂ et R₃, identiques ou différents, sont choisis parmi : l'hydrogène: un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (II), (III) ou (IV) dans lesquelles:
- B est un radical alkyle en C₁-C₄;
- R₆ est le radical méthyle.

3. Composition selon la revendication 1, **caractérisée par le fait que** le dérivé de 1,3,5-triazine est choisi parmi ceux présentant l'ensemble des caractéristiques suivantes :
- X₂ et X₃ sont identiques et représentent le radical.-NH-;
- R₃ est choisi parmi : un radical alkyle linéaire ou ramifié en C₁-C₁₈, un radical cycloalkyle en C₅-C₁₂ éventuellement substitué avec un ou plusieurs radicaux alkyles en C₁-C₄,
- R₁ est choisi parmi : l'hydrogène; un métal alcalin; un radical ammonium; un radical de formule (IV); un radical alkyle linéaire ou ramifié en C₁-C₁₈, un radical cycloalkyle en C₅-C₁₂ éventuellement substitué avec un ou plusieurs radicaux alkyles en C₁-C₄;
- R₂ est choisi parmi : un radical alkyle linéaire ou ramifié en C₁₋C₁₈; un radical cycloalkyle en C₅-C₁₂ éventuellement substitué avec un ou plusieurs radicaux alkyles en C₁-C₄.

4. Composition selon la revendication 1, **caractérisée par le fait que** le dérivé de 1,3,5-triazine est choisi parmi ceux présentant l'ensemble des caractéristiques suivantes :
- X₂ est l'oxygène;
- X₃ est le radical -NH-;
- R₃ est choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄;
- R₁ est choisi parmi : l'hydrogène; un métal alcalin; un radical ammonium; un radical de formule (IV); un radical alkyle linéaire ou ramifié en C₁-C₁₈; un radical cycloalkyle en C₅-C₁₂ éventuellement substitué avec un ou plusieurs radicaux alkyles en C₁₋C₄;
- R₂ est choisi parmi l'hydrogène; un métal alcalin; un radical ammonium; un radical de formule (IV); un radical alkyle linéaire ou ramifié en C₁-C₁₈; un radical cycloalkyle en C₅-C₁₂ éventuellement substitué avec un ou plusieurs radicaux alkyles en C₁-C₄.

5. Composition selon la revendication 4, **caractérisée par le fait que** le dérivé de 1,3,5-triazine est celui répondant à la formule suivante : 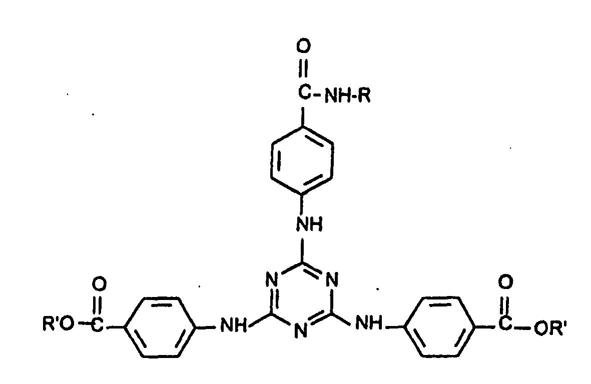 dans laquelle R' désigne un radical éthyl-2 hexyle et R désigne un radical ter. butyle.

6. Composition selon la revendication 1, **caractérisée par le fait que** le dérivé de 1.3.5-triazine est choisi parmi ceux présentant l'ensemble des caractéristiques suivantes :
- X₂ et X₃ sont identiques et représentent l'oxygène;
- R₁, R₂ et R₃ sont identiques et représentent un radical alkyle en C₆-C₁₂ ou un radical polyoxyéthyléné comprenant de 1 à 6 unités d'oxyde d'éthylène et dont le groupe OH terminal est méthylé.

7. Composition selon la revendication 6, **caractérisée par le fait que** le dérivé de 1,3,5-Mazine est celui répondant à la formule suivante : 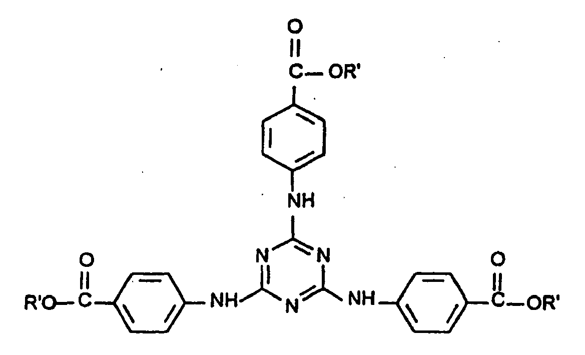 dans laquelle R' désigne un radical 2-éthyl hexyle.

8. Composition selon l'une quelconque des revendications 1 à 7, **caractérisée par le fait que** le dérivé de 1,3,5-triazine est présent dans la composition à une teneur allant de 0,5 % à 20 % en poids, par rapport au poids total de la composition.

9. Composition selon la revendication 8. **caractérisée par le fait que** ladite teneur va de 1 % à 10 % en poids, par rapport au poids total de la composition.

10. Composition selon l'une quelconque des revendications 1 à 9, **caractérisée par** le fait le β,β'-diphénylacrylate d'alkyle ou α-cyano-β,β'-diphénylacrylate d'alkyle est l'α-cyano-β,β' diphénylacrylate de 2-éthylhexyle.

11. Composition selon l'une quelconque des revendications 1 à 9, **caractérisée par** le fait le β,β'-dipnénylacrylate d'alkyle ou α-cyano-β,β'-diphénylacrylate d'alkyle est l'α-cyano-β,β' diphénylacrylate d'éthyle.

12. Composition selon l'une quelconque des revendications 1 à 11, **caractérisée par le fait que** le β,β'-dphénylacrylate d'alkyle ou α-cyano-β,β'-diphénylacrylate d'alkyle est présent dans la composition à une teneur au moins égale à 0,5 % en poids, par rapport au poids total de la composition.

13. Composition selon la revendication 12, **caractérisée par le fait que** ladite teneur va de 0,5 % à 20 % en poids, par rapport au poids total de la composition.

14. Composition selon l'une quelconque des revendications 1 à 13, **caractérisée par le fait que** le 4-tert-buryl-4'-métnoxydibenzoylméthane est présent dans la composition à une teneur allant de 0,2 % à 15 % en poids, par rapport au poids total de la composition.

15. Composition selon la revendication 14, **caractérisée par le fait que** ladite teneur va de 0,5 % à 10 % en poids, par rapport au poids total de la composition.

16. Composition selon l'une quelconque des revendications 1 à 15, **caractérisée par le fait qu'**elle est sous la forme d'une émulsion huile-dans-eau.

17. Utilisation d'un β,β'-diphénylacrylate d'alkyle ou α-cyano-β,β'-dlphénylacrylate d'alkyle dans, ou pour la fabrication de, compositions cosmétiques et/ou dermatologiques contenant le 4-tert-butyl-4'-méthcocydibenzoylméthane en association avec au moins un dérivé de 1,3,5-triazine tel que défini à l'une quelconque des revendications 1 à 7 en vue d'améliorer dans lesdites compositions la stabilité au rayonnement UV dudit dérivé de 1,3,5-triazine.

18. Utilisation selon la revendication 17, **caractérisée par le fait que** le β,β'-diphénylacrylate d'alkyle ou α-cyano-β,β'-diphénylacrylate d'alkyle est l'α-cyano-β,β' diphénylacrylate de 2-éthylhexyle.

19. Utilisation selon la revendication 17, **caractérisée par** le fait le β,β'-diphénylacrylate d'alkyle ou α-cyano-β,β'-diphénylacrylate d'alkyle est l'α-cyano-β,β' diphénytacrylate d'éthyle.

20. Utilisation selon l'une quelconque des revendications 17 à 19, **caractérisée par le fait que** le dérivé de 1,3,5-trtazine est la 2,4,8-tris[p-(2'-éthylhexyl-1'-oxycarbanyl)anilino]-1,3,5-triazine.

21. Utilisation selon l'une quelconque des revendications 17 à 20, **caractérisée par le fait que** le dérivé de 1,3,5-triazine est présent dans la composition à une teneur allant de 0,5 % à 20 % en poids, par rapport au poids total de la composition.

22. Utilisation selon la revendication 21, **caractérisée par le fait que** ladite teneur va de 1 % à 10 % en poids, par rapport au poids total de la composition.

23. Utilisation selon l'une quelconque des revendications 17 à 22, **caractérisée par le fait que** le β,β'-diphénylacrylate d'alkyle ou α-cyano-β,β'-diphérrylacrylate d'alkyle est présent dans la composition à une teneur au moins égale à 0,5 % en poids, par rapport au poids total de la composition.

24. Utilisation selon la revendication 23, **caractérisée par le fait que** ladite teneur va de 0,5 % à 20 % en poids, par rapport au poids total de la composition.

25. Utilisation selon l'une quelconque des revendications 17 à 24, **caractérisée par le fait que** le 4-tert-butyl-4'-méthoxydbenzoylméthane est présent dans la composition à une teneur allant de 0,2 % à 15 % en poids, par rapport au poids total de la composition.

26. Utilisation selon la revendication 25, **caractérisée par le fait que** ladite teneur va de 0,5 % à 10 % en poids, par rapport au poids total de la composition.

27. Procédé pour améliorer la stabilité au rayonnement UV des compositions cosmétiques et/ou dermatologique comprenant du 4-tert-butyl-4'-méthoxydbenzoylméthane et un dérivé de 1,3,5-triazine tel que défini à l'une quelconque des revendications 1 à 7, **caractérisé par le fait qu'**il consiste à introduire dans lesdites compositions une quantité efficace d'un β,β'-diphénylacrylate d'alkyle ou α-cyano-β,β'-diphénylacrylate d'alkyle.

28. Procédé selon la revendication 27, **caractérisé par le fait que** le β,β'-diphénylacrylate d'alkyle ou α-cyano-β,β'-diphénylacrylate d'alkyle est l'α-cyanc-β,β' diphénylacrylate de 2-éthylheocyle.

29. Procédé selon la revendication 27, **caractérisé par le fait que** le β,β'-diphénylacrylate d'alkyle ou α-cyano-β,β'-diphénylacrylate d'alkyle est l'α-cyano-β,β' diphénylacrylate d'éthyle.

30. Procédé selon l'une quelconque des revendications 27 à 29, **caractérisé par le fait que** le dérivé de 1,3,5-triazine est la 2,4,8-tris[p-(2'-éthylhexyl-1'-oxycarbonyl)anilino]-1,3,5-triazine.

31. Procédé de traitement cosmétique pour protéger la peau et/ou les cheveux contre le rayonnement UV, en particulier la rayonnement solaire, **caractérisé en ce qu'**il consiste à appliquer sur ces derniers une quantité efficace d'une composition cosmétique telle que à l'une quelconque des revendications 1 à 16.

## Revendications (Revendications pour l'(les) Etat(s) contractant(s) suivant(s): DE)

1. Composition cosmétique et/ou dermatologique comprenant, dans un support cosmétiquement et/ou dermatologiquement acceptable, i) du 4-tert-butyl-4'-méthoxydibanzoyl-méthane, ii) au moins un dérivé de 1,3.5-triazine répondant à la formule (I) suivante : 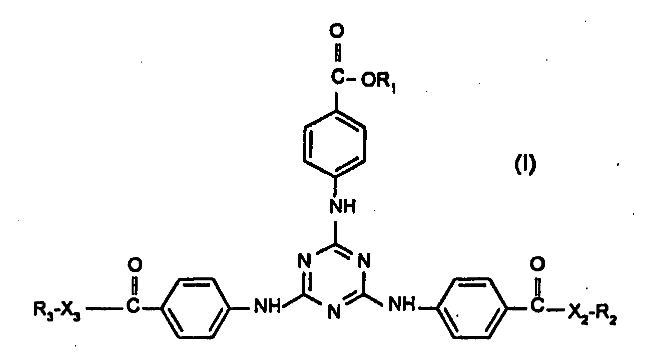 dans laquelle :
- X₂ et X₃, identiques ou différents, représentent l'oxygène ou le radical -NH-;
- R₁, R₂ et R₃, identiques ou différents, sont choisis parmi : l'hydrogène; un métal alcalin; un radical ammonium éventuellement substitué par un ou plusieurs radicaux alkyles ou hydroxyalkyles, un radical alkyle linéaire ou ramilié en C₁-C₁₈; un radical cycloalkyle en C₅-C₁₂ éventuellement substitué par un ou plusieurs radicaux alkyles en C₁-C₄; un radical polyoxyéthyléné comprenant de 1 à 6 unités d'oxyde d'éthylène et dont le groupe OH terminal est méthylé; un radical de formule (II), (III) ou (IV) suivantes : 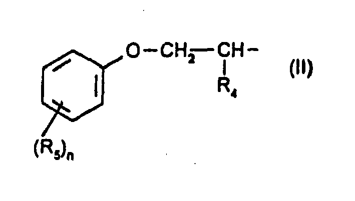 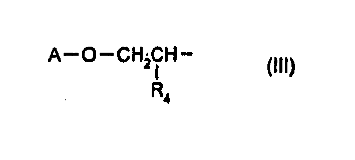 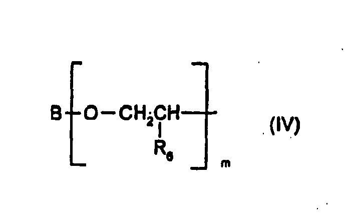
dans lesquelles:
- R₄ est l'hydrogène ou un radical méthyle;
- R₅ est un radical alkyle en C₁-C₉;
- n est un nombre entier allant de 0 à 3;
- m est un nombre entier allant de 1 à 10;
- A est un radical alkyle en C₄-C₈ ou un radical cycloalkyle en C₅-C₈,
- B est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄:
- R₆ est l'hydrogène ou un radical méthyle,
et iii) au moins un β,β'-diphénylacrylate d'alkyle ou α-cyano-β,β'-diphénylacrylate d'alkyle de formule (V) suivante : 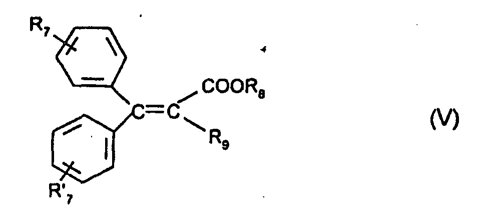
dans laquelle:
- R₇ et R'₇, identiques ou différents, sont en position méta ou para et sont choisis parmi : l'hydrogène; un radical alcoxy en C₁-C₈ à chaîne droite ou ramifiée; un radical alkyle en C₁-C₄ à chaîne droite ou ramifiée;
- R₈ représente un radical alkyle en C₁-C₁₂ à chaîne droite ou ramifiée;
- R₉ représente un atome d'hydrogène ou un radical -CN,
ladite composition étant exempte de p-méthoxycinnamate de 2-éthylhexyle **et sous réserve que la composition soit différente des formulations suivantes dans lesquelles les quantités des différents ingrédients sont indiquées en pourcentage en poids par rapport au poids total de la composition**
| **Composition 1** | |
|---|---|
| **Ingrédients** | **Quantités** |
| **Composant I, filtres UV** | |
| 4-Methylbenzylidene Camphor (Eusolex 6300) - Filtre UV-B | 3,0 |
| 2,4,6-tris[p-(2'-éthylhexyl-1'-oxycarbonyl)anilino]-1,3,5-triazine (Uvinul T150) - Filtre UV-B | 1,5 |
| 2-Ethylhexyl Salicylate (Neo Heliopan OS) Filtre UV-B | 4,0 |
| α-cyano-β,β' diphénylacrylate de 2-éthylhexyle (Neo Heliopan 303) Filtre UV-B | 4,0 |
| 4-tert-butyl-4'-méthoxydibenzoylméthane (Parsol 1789)- Filtre UV-A | 2,0 |
| **Composant II, composant gras** | |
|---|---|
| C₁₂-C₁₅ Alkyl Benzoate (Finsolv TN) | 12,0 |
| **Composant III** | |
|---|---|
| Huile parfum | 0,4 |
| Conservateur | q.s. |
| **Composant IV** | |
|---|---|
| Carbomer | 0,8 |
| Glycerine | 3,0 |
| EDTA | 0,1 |
| Eau | qsp 100 |
| **Composant V** | |
|---|---|
| Eau | 20,0 |
| Acide novantisolique (filtre UVB hydrosoluble) | 2,0 |
| Agent neutralisant (notamment NaOH, Triethanolamine | q.s |
| **Composition 2** | |
|---|---|
| **Ingrédients** | **Quantités** |
| **Composant I, filtres UV** | |
| 4-Methylbenzylidene Camphor (Eusolex 6300) - Filtre UV-B | 4,0 |
| 2,4,6-tris[p-(2'-éthylhexyl-1'-oxycarbonyl)anilino]-1,3,5-triazine (Uvinul T150) - Filtre UV-B | 2,0 |
| 2-Ethylhexyl Salicylate (Neo Heliopan OS) Filtre UV-B | 3,0 |
| α-cyano-β,β' diphénylacrylate de 2-éthylhexyle (Neo Heliopan 303) Filtre UV-B | 3,0 |
| 4-tert-butyl-4'-méthoxydibenzoylméthane (Parsol 1789) - Filtre UV-A | 3,0 |
| **Composant II, composant gras** | |
|---|---|
| C₁₂-C₁₅ Alkyl Benzoate - Finsolv TN | 12,0 |
| Caprylic/Capric Triglycerides (Miglyol 812) | 2,0 |
| Octyl Stearate (Cetiol 868) | 3,0 |
| **Composant III** | |
|---|---|
| Huile parfum | 0,4 |
| Conservateur | q.s. |
| **Composant IV** | |
|---|---|
| Carbomer | 0,4 |
| Glycerine | 3,0 |
| EDTA | 0,1 |
| Eau | qsp 100 |
| **Composant V** | |
|---|---|
| Agent neutralisant (notamment NaOH, Triethanolamine | q.s |

2. Composition selon la revendication 1, **caractérisée par le fait que** le dérivé de 1,3,5-triazine est choisi parmi ceux présentant l'ensemble des caractéristiques suivantes :
- X₂ et X₃ sont identiques et représentent l'oxygène:
- R₁ est choisi parmi : un radical cycloalkyle en C₅-C₁₂ éventuellement substitué par un ou plusieurs radicaux alkyles en C₁-C₄; un radical de formule (II), (III) ou (IV) dans lesquelles :
- B est un radical alkyle en C₁-C₄;
- R₆ est le radical méthyle;
- R₂ et R₃, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramitié en C₁-C₁₈; un radical cycloalkyle en C₅-C₁₂ éventuellement substitué par un ou plusieurs radicaux alkyles en C₁-C₄; un radical de formule (II), (III) ou (IV) dans lesquelles :
- B est un radical alkyle en C₁-C₄;
- R₆ est le radical méthyle.

3. Composition selon la revendication 1, **caractérisée par le fait que** le dérivé de 1,3,5-triazine est choisi parmi ceux présentant l'ensemble des caractéristiques suivantes :
- X₂ et X₃ sont identiques et représentent le radical -NH-;
- R₃ est choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄;
- R₁ est choisi parmi : l'hydrogène; un métal alcalin; un radical ammonium; un radical de formule (IV); un radical alkyle linéaire ou ramifié en C₁-C₁₈; un radical cycloalkyle en C₅-C₁₂ éventuellement substitué avec un ou plusieurs radicaux alkyles en C₁-C₄:
- R₂ est choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄.

4. Composition selon la revendication 1, **caractérisée par le fait que** le dérivé de 1,3.5-triazine est choisi parmi ceux présentant l'ensemble des caractéristiques suivantes :
- X₂ est l'oxygène;
- X₃ est le radical -NH-;
- R₃ est choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄;
- R₁ est choisi parmi : l'hydrogène; un métal alcalin; un radical ammonium; un radical de formule (IV): un radical alkyle linéaire ou ramifié en C₁-C₁₈; un radical cycloalkyle en C₅-C₁₂ éventuellement substitué avec un ou plusieurs radicaux alkyles en C₁-C₄;
- R₂ est choisi parmi l'hydrogène; un métal alcalin; un radical ammonium: un radical de formule (IV); un radical alkyle linéaire ou ramifié en C₁-C₁₈; un radical cycloalkyle en C₅-C₁₂ éventuellement substitué avec un ou plusieurs radicaux alkyles en C₁-C₄.

5. Composition selon la revendication 4, **caractérisée par le fait que** le dérivé de 1.3,5-triazine est celui répondant à la formule suivante : 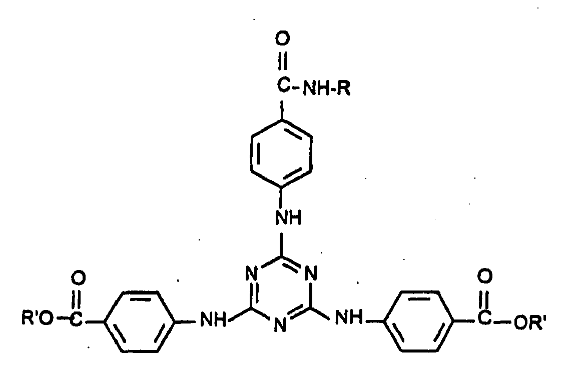 dans laquelle R' désigne un radical éthyl-2 hexyle et R désigne un radical ter. butyle.

6. Composition selon la revendication 1, **caractérisée par le fait que** le dérivé de 1,3,5-triazine est choisi parmi ceux présentant l'ensemble des caractéristiques suivantes :
- X₂ et X₃ sont identiques et représentent l'oxygène;
- R₁, R₂ et R₃ sont identiques et représentent un radical alkyle en C₆-C₁₂ ou un radical polyoxyéthyléné comprenant de 1 à 6 unités d'oxyde d'éthylène et dont le groupe OH terminal est méthylé.

7. Composition selon la revendication 6, **caractérisée par le fait que** le dérivé de 1,3,5-triazine est celui répondant à la formule suivante : 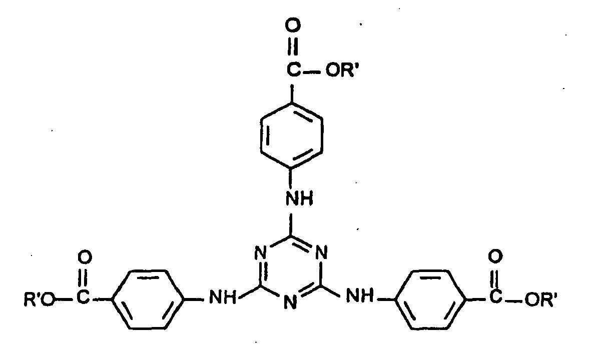 dans laquelle R' désigne un radical 2-éthyl hexyle.

8. Composition selon l'une quelconque des revendications 1 à 7, **caractérisée par le fait que** le dérivé de 1,3,5-triazine est présent dans la composition à une teneur allant de 0,5 % à 20 % en poids, par rapport au poids total de la composition.

9. Composition selon la revendication 8, **caractérisée par le fait que** ladite teneur va de 1 % à 10 % en poids, par rapport au poids total de la composition.

10. Composition selon l'une quelconque des revendications 1 à 9, **caractérisée par** le fait le β,β'-diphénylacrylate d'alkyle ou α-cyano-β,β'-diphénylacrylate d'alkyle est l'α-cyano-β,β' diphénylacrylate de 2-éthylhexyle.

11. Composition selon l'une quelconque des revendications 1 à 9, **caractérisée par** le fait le β,β'-diphénylacrylate d'alkyle ou α-cyano-β,β'-diphénylacrylate d'alkyle est l'α-cyano-β,β' diphénylacrylate d'éthyle.

12. Composition selon l'une quelconque des revendications 1 à 11, **caractérisée par le fait que** le β,β'-diphénylacrylate d'alkyle ou α-cyano-β,β'-diphénylacrylate d'alkyle est présent dans la composition à une teneur au moins égale à 0,5 % en poids, par rapport au poids total de la composition.

13. Composition selon la revendication 12, **caractérisée par le fait que** ladite teneur va de 0,5 % à 20 % en poids, par rapport au poids total de la composition.

14. Composition selon l'une quelconque des revendications 1 à 13, **caractérisée par le fait que** le 4-tert-butyl-4'-méthoxydibenzoylméthane est présent dans la composition à une teneur allant de 0,2 % à 15 % en poids, par rapport au poids total de la composition.

15. Composition selon la revendication 14, **caractérisée par le fait que** ladite teneur va de 0,5 % à 10 % en poids, par rapport au poids total de la composition.

16. Composition selon l'une quelconque des revendications 1 à 15, **caractérisée par le fait qu'**elle est sous la forme d'une émulsion huile-dans-eau.

17. Utilisation d'un β,β'-diphénylacrylate d'alkyle ou α-cyano-β,β'-diphénylacrylate d'alkyle dans, ou pour la fabrication de, compositions cosmétiques et/ou dermatologiques contenant le 4-tert-butyl-4'-méthoxydibenzoylméthane en association avec au moins un dérivé de 1,3,5-triazine tel que défini à l'une quelconque des revendications 1 à 7 en vue d'améliorer dans lesdites compositions la stabilité au rayonnement UV dudit dérivé de 1,3,5-triazine.

18. Utilisation selon la revendication 17, **caractérisée par le fait que** le β,β'-diphénylacrylate d'alkyle ou α-cyano-β,β'-diphénylacrylate d'alkyle est l'α-cyano-β,β' diphénylacrylate de 2-éthylhexyle.

19. Utilisation selon la revendication 17, **caractérisée par** le fait le β,β'-diphénylacrylate d'alkyle ou α-cyano-β,β'-diphénylacrylate d'alkyle est l'α-cyano-β,β' diphénylacrylate d'éthyle.

20. Utilisation selon l'une quelconque des revendications 17 à 19, **caractérisée par le fait que** le dérivé de 1,3,5-triazine est la 2,4,6-tris[p-(2'-éthylhexyl-1'-oxycarbonyl)anilino]-1,3,5-triazine.

21. Utilisation selon l'une quelconque des revendications 17 à 20, **caractérisée par le fait que** le dérivé de 1,3,5-triazine est présent dans la composition à une teneur allant de 0,5 % à 20 % en poids, par rapport au poids total de la composition.

22. Utilisation selon la revendication 21, **caractérisée par le fait que** ladite teneur va de 1 % à 10 % en poids, par rapport au poids total de la composition.

23. Utilisation selon l'une quelconque des revendications 17 à 22, **caractérisée par le fait que** le β,β'-diphénylacrylate d'alkyle ou α-cyano-β,β'-diphénylacrylate d'alkyle est présent dans la composition à une teneur au moins égale à 0,5 % en poids, par rapport au poids total de la composition.

24. Utilisation selon la revendication 23, **caractérisée par le fait que** ladite teneur va de 0.5 % à 20 % en poids, par rapport au poids total de la composition.

25. Utilisation selon l'une quelconque des revendications 17 à 24, **caractérisée par le fait que** le 4-tert-butyl-4'-méthoxydbenzoylméthane est présent dans la composition à une teneur allant de 0,2 % à 15 % en poids, par rapport au poids total de la composition.

26. Utilisation selon la revendication 25, **caractérisé par le fait que** ladite teneur va de 0,5 % à 10 % en poids, par rapport au poids total de la composition.

27. Procédé pour améliorer la stabilité au rayonnement UV des compositions cosmétiques et/ou dermatologique comprenant du 4-tert-butyl-4'-méthoxydibenzoylméthane et un dérivé de 1,3,5-triazine tel que défini à l'une quelconque des revendications 1 à 7, **caractérisé par le fait qu'**il consiste à introduire dans lesdites compositions une quantité efficace d'un β,β'-diphénylacrylate d'alkyle ou α-cyano-β,β'-diphénylacrylate d'alkyle.

28. Procédé selon la revendication 27, **caractérisé par le fait que** le β,β'-diphénylacrylate d'alkyle ou α-cyano-β,β'-diphénylacrylate d'alkyle est l'α-cyano-β,β' diphénylacrylate de 2-éthylhexyle.

29. Procédé selon la revendication 27, **caractérisé par le fait que** le β,β'-diphénylacrylate d'alkyle ou α-cyano-β,β'-diphénylacrylate d'alkyle est l'α-cyano-β,β' diphénylacrylate d'éthyle.

30. Procédé selon l'une quelconque des revendications 27 à 29, **caractérisé par le fait que** le dérivé de 1,3,5-triazine est la 2,4,6-tris[p-(2'-éthylhexyl-1'-oxycarbonyl)amilino]-1,3,5-triazine.

31. Procédé de traitement cosmétique pour protéger la peau et/ou les cheveux contre le rayonnement UV, en particulier le rayonnement solaire, **caractérisé en ce qu'**il consiste à appliquer sur ces derniers une quantité efficace d'une composition cosmétique telle que à l'une quelconque des revendications 1 à 16.

## Claims (Claims for the following Contracting State(s): ES, GB, FR, IT)

1. Cosmetic and/or dermatological composition including, in a cosmetically and/or dermatologically acceptable carrier, i) 4-tert-butyl-4'-methoxydibenzoylmethane, ii) at least one 1,3,5-triazine derivative corresponding to the following formula (I): 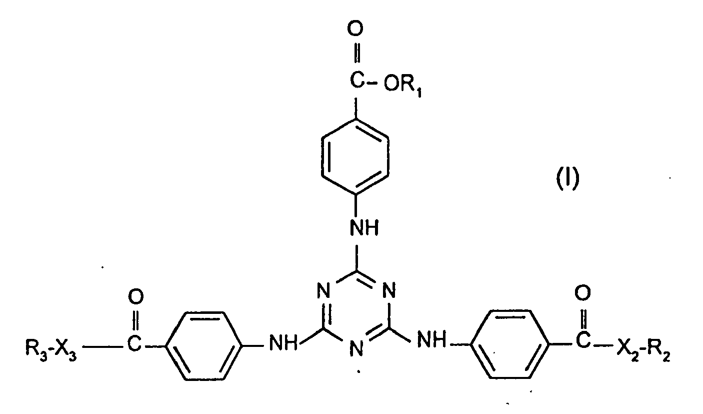 in which:
- X₂ and X₃, which are identical or different, denote oxygen or the -NH- radical,
- R₁, R₂ and R₃, which are identical or different, are chosen from hydrogen, an alkali metal, an ammonium radical optionally substituted by one or more alkyl or hydroxyalkyl radicals, a linear or branched C₁-C₁₈ alkyl radical, a C₅-C₁₂ cycloalkyl radical optionally substituted by one or more C₁-C₄ alkyl radicals, a polyoxyethylenated radical including from 1 to 6 ethylene oxide units and in which the end OH group is methylated, a radical of the following formulae (II), (III) and (IV): 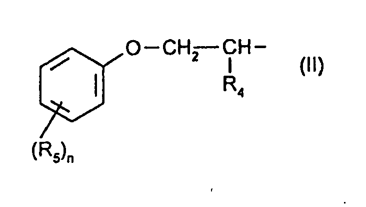 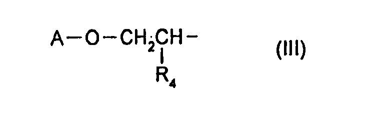 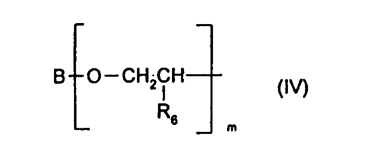
in which:
- R₄ is hydrogen or a methyl radical,
- R₅ is a C₁-C₉ alkyl radical,
- n is an integer ranging from 0 to 3,
- m is an integer ranging from 1 to 10,
- A is a C₄-C₈ alkyl radical or a C₅-C₈ cycloalkyl radical,
- B is chosen from a linear or branched C₁-C₈ alkyl radical, a C₅-C₈ cycloalkyl radical and an aryl radical optionally substituted by one or more C₁-C₄ alkyl radicals,
- R₆ is hydrogen or a methyl radical,
and iii) at least one alkyl β,β'-diphenylacrylate or alkyl α-cyano-β,β'-diphenylacrylate of the following formula (V): 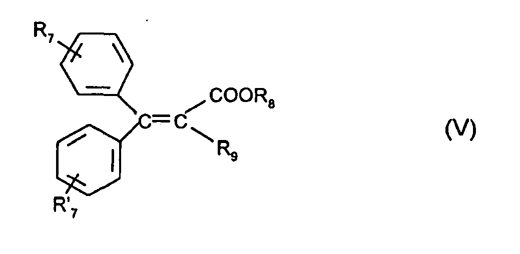
in which:
- R₇ and R'₇, which are identical or different, are in a meta or para position and are chosen from hydrogen, a C₁-C₈ alkoxy radical with a straight or branched chain and a C₁-C₄ alkyl radical with a straight or branched chain,
- R₈ denotes a C₁-C₁₂ alkyl radical with a straight or branched chain,
- R₉ denotes a hydrogen atom or a -CN radical,
said composition being free of 2-ethylhexyl p-methoxycinnamate **and with the proviso that the composition is different from the following formulation**
**in which the amounts of the various ingredients are indicated as percentage by weight relative to the total weight of the composition**
| **Ingredients** | **Amounts** |
|---|---|
| **Component I, UV screens** | |
| 4-Methylbenzylidenecamphor (Eusolex 6300) - UV-B screen | 3.0 |
| 2,4,6-tris[p-(2'-Ethylhexyl-1'-oxycarbonyl)-anilino]-1,3,5-triazine (Uvinul T150) - UV-B screen | 1.5 |
| 2-Ethylhexyl salicylate (Neo Heliopan OS) - UV-B screen | 4.0 |
| 2-Ethylhexyl α-cyano-β,β'-diphenylacrylate (Neo Heliopan 303) - UV-B screen | 4.0 |
| 4-tert-Butyl-4'-methoxydibenzoylmethane (Parsol 1789) - UV-A screen | 2.0 |
| **Component II, fatty component** | |
|---|---|
| C₁₂-C₁₅ Alkyl Benzoate (Finsolv TN) | 12.0 |
| **Component III** | |
|---|---|
| Fragrance oil | 0.4 |
| Preservative | q.s. |
| **Component IV** | |
|---|---|
| Carbomer | 0.8 |
| Glycerol | 3.0 |
| EDTA | 0.1 |
| Water | q.s. 100 |
| **Component V** | |
|---|---|
| Water | 20.0 |
| Novantisolic acid (water-soluble UV-B screen) | 2.0 |
| Neutralizing agent (in particular NaOH, triethanolamine) | q.s. |

2. Composition according to Claim 1, **characterized in that** the 1,3,5-triazine derivative is chosen from those exhibiting all of the following characteristics:
- X₂ and X₃ are identical and denote oxygen,
- R₁ is chosen from a C₅-C₁₂ cycloalkyl radical optionally substituted by one or more C₁-C₄ alkyl radicals, a radical of formula (II), (III) or (IV), in which:
- B is a C₁-C₄ alkyl radical,
- R₆ is the methyl radical,
- R₂ and R₃, which are identical or different, are chosen from hydrogen, an alkali metal, an ammonium radical optionally substituted by one or more alkyl or hydroxyalkyl radicals, a linear or branched C₁-C₁₈ alkyl radical, a C₅-C₁₂ cycloalkyl radical optionally substituted by one or more C₁-C₄ alkyl radicals, and a radical of formula (II), (III) or (IV) in which:
- B is a C₁-C₄ alkyl radical,
- R₆ is the methyl radical.

3. Composition according to Claim 1, **characterized in that** the 1,3,5-triazine derivative is chosen from those exhibiting all of the following characteristics:
- X₂ and X₃ are identical and denote the -NH-radical,
- R₃ is chosen from a linear or branched C₁-C₁₈ alkyl radical and a C₅-C₁₂ cycloalkyl radical optionally substituted by one or more C₁-C₄ alkyl radicals,
- R₁ is chosen from hydrogen, an alkali metal, an ammonium radical, a radical of formula (IV), a C₁-C₁₈ linear or branched alkyl radical and a C₅-C₁₂ cycloalkyl radical optionally substituted by one or more C₁-C₄ alkyl radicals,
- R₂ is chosen from a C₁-C₁₈ linear or branched alkyl radical and a C₅-C₁₂ cycloalkyl radical optionally substituted by one or more C₁-C₄ alkyl radicals.

4. Composition according to Claim 1, **characterized in that** the 1,3,5-triazine derivative is chosen from those exhibiting all of the following characteristics:
- X₂ is oxygen,
- X₃ is the -NH- radical,
- R₃ is chosen from a C₁-C₁₈ linear or branched alkyl radical and a C₅-C₁₂ cycloalkyl radical optionally substituted by one or more C₁-C₄ alkyl radicals,
- R₁ is chosen from hydrogen, an alkali metal, an ammonium radical, a radical of formula (IV), a linear or branched C₁-C₁₈ alkyl radical and a C₅-C₁₂ cycloalkyl radical optionally substituted by one or more C₁-C₄ alkyl radicals,
- R₂ is chosen from hydrogen, an alkali metal, an ammonium radical, a radical of formula (IV), a linear or branched C₁-C₁₈ alkyl radical and a C₅-C₁₂ cycloalkyl radical optionally substituted by one or more C₁-C₄ alkyl radicals.

5. Composition according to Claim 4, **characterized in that** the 1,3,5-triazine derivative is that corresponding to the following formula: 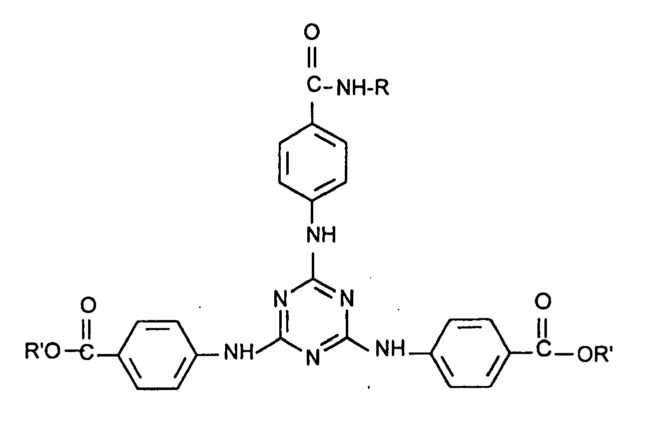 in which R' denotes a 2-ethylhexyl radical and R denotes a tert-butyl radical.

6. Composition according to Claim 1, **characterized in that** the 1,3,5-triazine derivative is chosen from those exhibiting all of the following characteristics:
- X₂ and X₃ are identical and denote oxygen,
- R₁, R₂ and R₃ are identical and denote a C₆-C₁₂ alkyl radical or a polyoxyethylene radical including from 1 to 6 ethylene oxide units and in which the end OH group is methylated.

7. Composition according to Claim 6, **characterized in that** the 1,3,5-triazine derivative is that corresponding to the following formula: 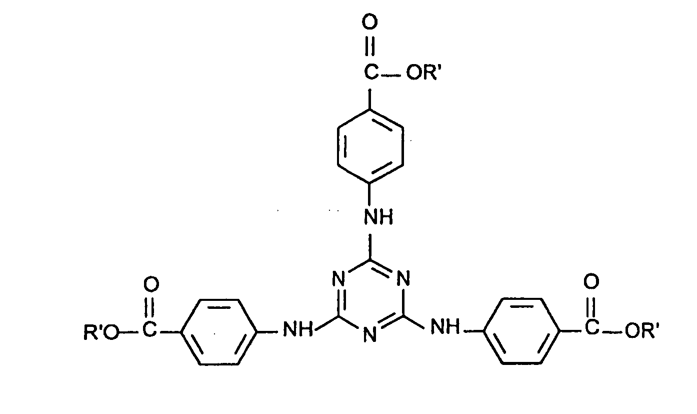 in which R' denotes a 2-ethylhexyl radical.

8. Composition according to any one of Claims 1 to 7, **characterized in that** the 1,3,5-triazine derivative is present in the composition in a content ranging from 0.5% to 20% by weight relative to the total weight of the composition.

9. Composition according to Claim 8, **characterized in that** the said content ranges from 1% to 10% by weight relative to the total weight of the composition.

10. Composition according to any one of Claims 1 to 9, **characterized in that** the alkyl β,β'-diphenylacrylate or alkyl α-cyano-β,β'-diphenylacrylate is 2-ethylhexyl α-cyano-β,β'-diphenylacrylate.

11. Composition according to any one of Claims 1 to 9, **characterized in that** the alkyl β,β'-diphenylacrylate or alkyl α-cyano-β,β'-diphenylacrylate is ethyl α-cyano-β,β'-diphenylacrylate.

12. Composition according to any one of Claims 1 to 11, **characterized in that** the alkyl β,β'-diphenylacrylate or alkyl α-cyano-β,β'-diphenylacrylate is present in the composition in a content of at least 0.5% by weight relative to the total weight of the composition.

13. Composition according to Claim 12, **characterized in that** the said content ranges from 0.5% to 20% by weight relative to the total weight of the composition.

14. Composition according to any one of Claims 1 to 13, **characterized in that** 4-tert-butyl-4'-methoxydibenzoylmethane is present in the composition in a content ranging from 0.2% to 15% by weight relative to the total weight of the composition.

15. Composition according to Claim 14, **characterized in that** the said content ranges from 0.5% to 10% by weight relative to the total weight of the composition.

16. Composition according to any one of Claims 1 to 15, **characterized in that** it is in the form of an oil-in-water emulsion.

17. Use of an alkyl β,β'-diphenylacrylate or alkyl α-cyano-β,β'-diphenylacrylate in, or for the manufacture of, cosmetic and/or dermatological compositions containing 4-tert-butyl-4'-methoxydibenzoylmethane in combination with at least one 1,3,5-triazine derivative as defined in any one of Claims 1 to 7, with a view to improving the UV radiation stability of the said 1,3,5-triazine derivative in the said compositions.

18. Use according to Claim 17, **characterized in that** the alkyl β,β'-diphenylacrylate or alkyl α-cyano-β,β'-diphenylacrylate is 2-ethylhexyl α-cyano-β,β'-diphenylacrylate.

19. Use according to Claim 17, **characterized in that** the alkyl β,β'-diphenylacrylate or alkyl α-cyano-β,β'-diphenylacrylate is ethyl α-cyano-β,β'-diphenylacrylate.

20. Use according to any one of Claims 17 to 19, **characterized in that** the 1,3,5-triazine derivative is 2,4,6-tris[p-(2'-ethylhexyl-1'-oxycarbonyl)anilino]-1,3,5-triazine.

21. Use according to any one of Claims 17 to 20, **characterized in that** the 1,3,5-triazine derivative is present in the composition in a content ranging from 0.5% to 20% by weight relative to the total weight of the composition.

22. Use according to Claim 21, **characterized in that** the said content ranges from 1% to 10% by weight relative to the total weight of the composition.

23. Use according to any one of Claims 17 to 22, **characterized in that** the alkyl β,β'-diphenylacrylate or alkyl α-cyano-β,β'-diphenylacrylate is present in the composition in a content of at least 0.5% by weight relative to the total weight of the composition.

24. Use according to Claim 23, **characterized in that** the said content ranges from 0.5% to 20% by weight relative to the total weight of the composition.

25. Use according to any one of Claims 17 to 24, **characterized in that** 4-tert-butyl-4'-methoxydibenzoylmethane is present in the composition in a content ranging from 0.2% to 15% by weight relative to the total weight of the composition.

26. Use according to Claim 25, **characterized in that** the said content ranges from 0.5% to 10% by weight relative to the total weight of the composition.

27. Process for improving the stability to UV radiation of cosmetic and/or dermatological compositions including 4-tert-butyl-4'-methoxydibenzoylmethane and a 1,3,5-triazine derivative as defined in any one of Claims 1 to 7, **characterized in that** it consists in introducing into the said compositions an effective quantity of an alkyl β,β'-diphenylacrylate or alkyl α-cyano-β,β'-diphenylacrylate.

28. Process according to Claim 27, **characterized in that** the alkyl β,β'-diphenylacrylate or alkyl α-cyano-β,β'-diphenylacrylate is 2-ethylhexyl α-cyano-β,β'-diphenylacrylate.

29. Process according to Claim 27, **characterized in that** the alkyl β,β'-diphenylacrylate or alkyl α-cyano-β,β'-diphenylacrylate is ethyl α-cyano-β,β'-diphenylacrylate.

30. Process according to any one of Claims 27 to 29, **characterized in that** the 1,3,5-triazine derivative is 2,4,6-tris[p-(2'-ethylhexyl-1'-oxycarbonyl)anilino]-1,3,5-triazine.

31. Process of cosmetic treatment for protecting the skin and/or the hair against UV radiation, in particular solar radiation, **characterized in that** it consists in applying thereto an effective quantity of a cosmetic composition such as in any one of Claims 1 to 16.

## Claims (Claims for the following Contracting State(s): DE)

1. Cosmetic and/or dermatological composition including, in a cosmetically and/or dermatologically acceptable carrier, i) 4-tert-butyl-4'-methoxydibenzoylmethane, ii) at least one 1,3,5-triazine derivative corresponding to the following formula (I): 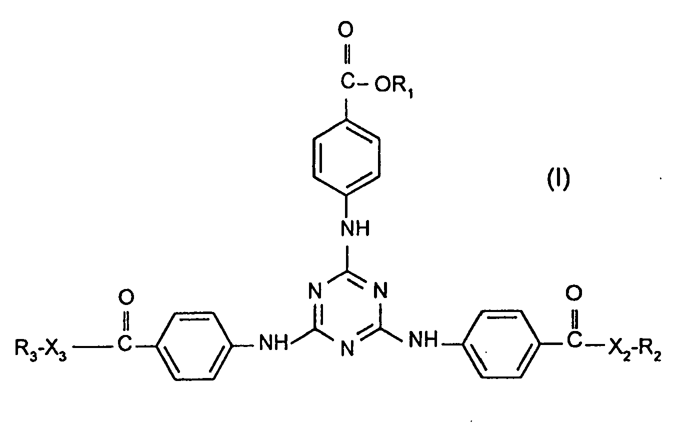 in which:
- X₂ and X₃, which are identical or different, denote oxygen or the -NH- radical,
- R₁, R₂ and R₃, which are identical or different, are chosen from hydrogen, an alkali metal, an ammonium radical optionally substituted by one or more alkyl or hydroxyalkyl radicals, a linear or branched C₁-C₁₈ alkyl radical, a C₅-C₁₂ cycloalkyl radical optionally substituted by one or more C₁-C₄ alkyl radicals, a polyoxyethylenated radical including from 1 to 6 ethylene oxide units and in which the end OH group is methylated, a radical of the following formulae (II), (III) and (IV): 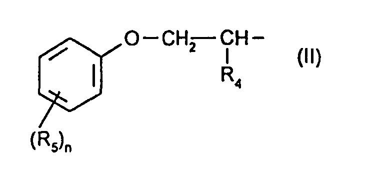 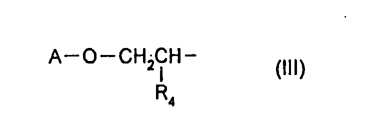 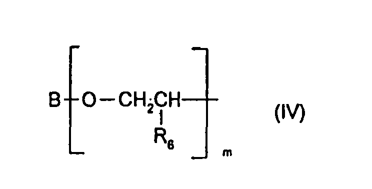
in which:
- R₄ is hydrogen or a methyl radical,
- R₅ is a C₁-C₉ alkyl radical,
- n is an integer ranging from 0 to 3,
- m is an integer ranging from 1 to 10,
- A is a C₄-C₈ alkyl radical or a C₅-C₈ cycloalkyl radical,
- B is chosen from a linear or branched C₁-C₈ alkyl radical, a C₅-C₈ cycloalkyl radical and an aryl radical optionally substituted by one or more C₁-C₄ alkyl radicals,
- R₆ is hydrogen or a methyl radical,
and iii) at least one alkyl β,β'-diphenylacrylate or alkyl α-cyano-β,β'-diphenylacrylate of the following formula (V): 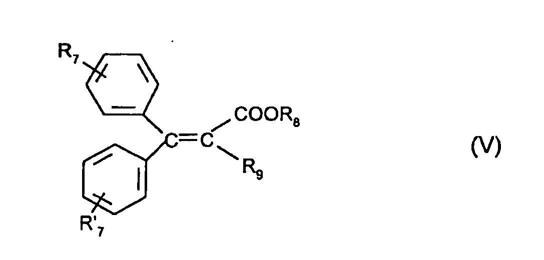
in which:
- R₇ and R'₇, which are identical or different, are in a meta or para position and are chosen from hydrogen, a C₁-C₈ alkoxy radical with a straight or branched chain and a C₁-C₄ alkyl radical with a straight or branched chain,
- R₈ denotes a C₁-C₁₂ alkyl radical with a straight or branched chain,
- R₉ denotes a hydrogen atom or a -CN radical,
said composition being free of 2-ethylhexyl p-methoxycinnamate **and with the proviso that the composition is different from the following formulations in which the amounts of the various ingredients are indicated as percentage by weight relative to the total weight of the composition**
| **Composition 1** | |
|---|---|
| **Ingredients** | **Amounts** |
| **Component I, UV screens** | |
| 4-Methylbenzylidenecamphor (Eusolex 6300) - UV-B screen | 3.0 |
| 2,4,6-tris[p-(2'-Ethylhexyl-1'-oxycarbonyl)-anilino]-1,3,5-triazine (Uvinul T150) - UV-B screen | 1.5 |
| 2-Ethylhexyl salicylate (Neo Heliopan OS) - UV-B screen | 4.0 |
| 2-Ethylhexyl α-cyano-β,β'-diphenylacrylate (Neo Heliopan 303) - UV-B screen | 4.0 |
| **Ingredients** | **Amounts** |
|---|---|
| 4-tert-Butyl-4'-methoxydibenzoylmethane (Parsol 1789) - UV-A screen | 2.0 |
| **Component II, fatty component** | |
|---|---|
| C₁₂-C₁₅ Alkyl Benzoate (Finsolv TN) | 12.0 |
| **Component III** | |
|---|---|
| Fragrance oil | 0.4 |
| Preservative | q.s. |
| **Component IV** | |
|---|---|
| Carbomer | 0.8 |
| Glycerol | 3.0 |
| EDTA | 0.1 |
| Water | q.s. 100 |
| **Component V** | |
|---|---|
| Water | 20.0 |
| Novantisolic acid (water-soluble UV-B screen) | 2.0 |
| Neutralizing agent (in particular NaOH, triethanolamine) | q.s. |
| **Composition 2** | |
|---|---|
| **Ingredients** | **Amounts** |
| **Component I, UV screens** | |
| 4-Methylbenzylidenecamphor (Eusolex 6300) - UV-B screen | 4.0 |
| 2,4,6-tris[p-(2'-Ethylhexyl-1'-oxycarbonyl)-anilino]-1,3,5-triazine (Uvinul T150) - UV-B screen | 2.0 |
| 2-Ethylhexyl salicylate (Neo Heliopan OS) - UV-B screen | 3.0 |
| 2-Ethylhexyl α-cyano-β,β'-diphenylacrylate (Neo Heliopan 303) - UV-B screen | 3.0 |
| 4-tert-Butyl-4'-methoxydibenzoylmethane (Parsol 1789) - UV-A screen | 3.0 |
| **Component II, fatty component** | |
|---|---|
| C₁₂-C₁₅ Alkyl Benzoate (Finsolv TN) | 12.0 |
| Caprylic/Capric Triglycerides (Miglyol 812) | 2.0 |
| **Ingredients** | **Amounts** |
|---|---|
| Octyl Stearate (Cetiol 868) | 3.0 |
| **Component III** | |
|---|---|
| Fragrance oil | 0.4 |
| Preservative | q.s. |
| **Component IV** | |
|---|---|
| Carbomer | 0.4 |
| Glycerol | 3.0 |
| EDTA | 0.1 |
| Water | q.s. 100 |
| **Component V** | |
|---|---|
| Neutralizing agent (in particular NaOH, triethanolamine) | q.s. |

2. Composition according to Claim 1, **characterized in that** the 1,3,5-triazine derivative is chosen from those exhibiting all of the following characteristics:
- X₂ and X₃ are identical and denote oxygen,
- R₁ is chosen from a C₅-C₁₂ cycloalkyl radical optionally substituted by one or more C₁-C₄ alkyl radicals, a radical of formula (II), (III) or (IV), in which:
- B is a C₁-C₄ alkyl radical,
- R₆ is the methyl radical,
- R₂ and R₃, which are identical or different, are chosen from hydrogen, an alkali metal, an ammonium radical optionally substituted by one or more alkyl or hydroxyalkyl radicals, a linear or branched C₁-C₁₈ alkyl radical, a C₅-C₁₂ cycloalkyl radical optionally substituted by one or more C₁-C₄ alkyl radicals, and a radical of formula (II), (III) or (IV) in which:
- B is a C₁-C₄ alkyl radical,
- R₆ is the methyl radical.

3. Composition according to Claim 1, **characterized in that** the 1,3,5-triazine derivative is chosen from those exhibiting all of the following characteristics:
- X₂ and X₃ are identical and denote the -NH-radical,
- R₃ is chosen from a linear or branched C₁-C₁₈ alkyl radical and a C₅-C₁₂ cycloalkyl radical optionally substituted by one or more C₁-C₄ alkyl radicals,
- R₁ is chosen from hydrogen, an alkali metal, an ammonium radical, a radical of formula (IV), a C₁-C₁₈ linear or branched alkyl radical and a C₅-C₁₂ cycloalkyl radical optionally substituted by one or more C₁-C₄ alkyl radicals,
- R₂ is chosen from a C₁-C₁₈ linear or branched alkyl radical and a C₅-C₁₂ cycloalkyl radical optionally substituted by one or more C₁-C₄ alkyl radicals.

4. Composition according to Claim 1, **characterized in that** the 1,3,5-triazine derivative is chosen from those exhibiting all of the following characteristics:
- X₂ is oxygen,
- X₃ is the -NH- radical,
- R₃ is chosen from a C₁-C₁₈ linear or branched alkyl radical and a C₅-C₁₂ cycloalkyl radical optionally substituted by one or more C₁-C₄ alkyl radicals,
- R₁ is chosen from hydrogen, an alkali metal, an ammonium radical, a radical of formula (IV), a linear or branched C₁-C₁₈ alkyl radical and a C₅-C₁₂ cycloalkyl radical optionally substituted by one or more C₁-C₄ alkyl radicals,
- R₂ is chosen from hydrogen, an alkali metal, an ammonium radical, a radical of formula (IV), a linear or branched C₁-C₁₈ alkyl radical and a C₅-C₁₂ cycloalkyl radical optionally substituted by one or more C₁-C₄ alkyl radicals.

5. Composition according to Claim 4, **characterized in that** the 1,3,5-triazine derivative is that corresponding to the following formula: 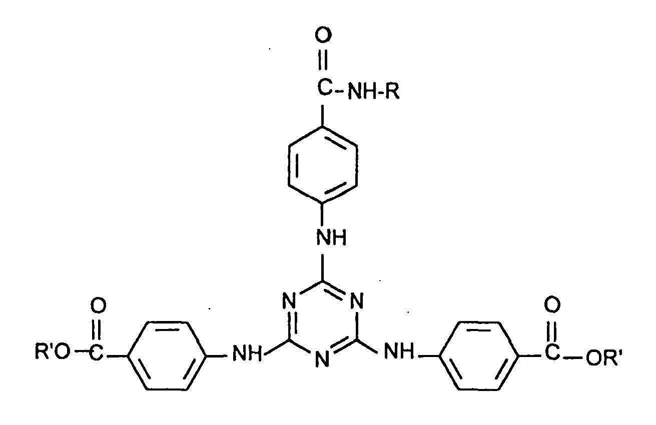 in which R' denotes a 2-ethylhexyl radical and R denotes a tert-butyl radical.

6. Composition according to Claim 1, **characterized in that** the 1,3,5-triazine derivative is chosen from those exhibiting all of the following characteristics:
- X₂ and X₃ are identical and denote oxygen,
- R₁, R₂ and R₃ are identical and denote a C₆-C₁₂ alkyl radical or a polyoxyethylene radical including from 1 to 6 ethylene oxide units and in which the end OH group is methylated.

7. Composition according to Claim 6, **characterized in that** the 1,3,5-triazine derivative is that corresponding to the following formula: 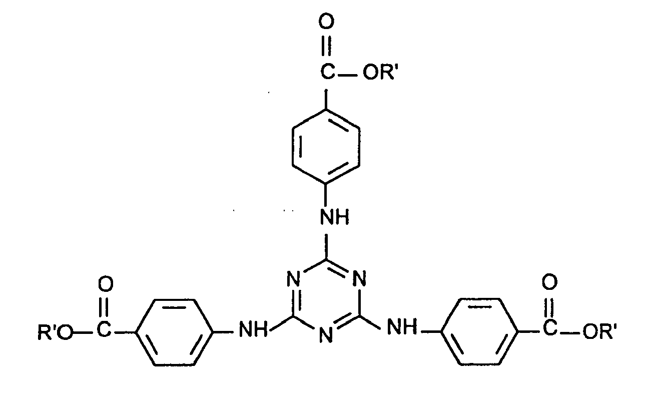 in which R' denotes a 2-ethylhexyl radical.

8. Composition according to any one of Claims 1 to 7, **characterized in that** the 1,3,5-triazine derivative is present in the composition in a content ranging from 0.5% to 20% by weight relative to the total weight of the composition.

9. Composition according to Claim 8, **characterized in that** the said content ranges from 1% to 10% by weight relative to the total weight of the composition.

10. Composition according to any one of Claims 1 to 9, **characterized in that** the alkyl β,β'-diphenylacrylate or alkyl α-cyano-β,β'-diphenylacrylate is 2-ethylhexyl α-cyano-β,β'-diphenylacrylate.

11. Composition according to any one of Claims 1 to 9, **characterized in that** the alkyl β,β'-diphenylacrylate or alkyl α-cyano-β,β'-diphenylacrylate is ethyl α-cyano-β,β'-diphenylacrylate.

12. Composition according to any one of Claims 1 to 11, **characterized in that** the alkyl β,β'-diphenylacrylate or alkyl α-cyano-β,β'-diphenylacrylate is present in the composition in a content of at least 0.5% by weight relative to the total weight of the composition.

13. Composition according to Claim 12, **characterized in that** the said content ranges from 0.5% to 20% by weight relative to the total weight of the composition.

14. Composition according to any one of Claims 1 to 13, **characterized in that** 4-tert-butyl-4'-methoxydibenzoylmethane is present in the composition in a content ranging from 0.2% to 15% by weight relative to the total weight of the composition.

15. Composition according to Claim 14, **characterized in that** the said content ranges from 0.5% to 10% by weight relative to the total weight of the composition.

16. Composition according to any one of Claims 1 to 15, **characterized in that** it is in the form of an oil-in-water emulsion.

17. Use of an alkyl β,β'-diphenylacrylate or alkyl α-cyano-β,β'-diphenylacrylate in, or for the manufacture of, cosmetic and/or dermatological compositions containing 4-tert-butyl-4'-methoxydibenzoylmethane in combination with at least one 1,3,5-triazine derivative as defined in any one of Claims 1 to 7, with a view to improving the UV radiation stability of the said 1,3,5-triazine derivative in the said compositions.

18. Use according to Claim 17, **characterized in that** the alkyl β,β'-diphenylacrylate or alkyl α-cyano-β,β'-diphenylacrylate is 2-ethylhexyl α-cyano-β,β'-diphenylacrylate.

19. Use according to Claim 17, **characterized in that** the alkyl β,β'-diphenylacrylate or alkyl α-cyano-β,β'-diphenylacrylate is ethyl α-cyano-β,β'-diphenylacrylate.

20. Use according to any one of Claims 17 to 19, **characterized in that** the 1,3,5-triazine derivative is 2,4,6-tris[p-(2'-ethylhexyl-1'-oxycarbonyl)anilino]-1,3,5-triazine.

21. Use according to any one of Claims 17 to 20, **characterized in that** the 1,3,5-triazine derivative is present in the composition in a content ranging from 0.5% to 20% by weight relative to the total weight of the composition.

22. Use according to Claim 21, **characterized in that** the said content ranges from 1% to 10% by weight relative to the total weight of the composition.

23. Use according to any one of Claims 17 to 22, **characterized in that** the alkyl β,β'-diphenylacrylate or alkyl α-cyano-β,β'-diphenylacrylate is present in the composition in a content of at least 0.5% by weight relative to the total weight of the composition.

24. Use according to Claim 23, **characterized in that** the said content ranges from 0.5% to 20% by weight relative to the total weight of the composition.

25. Use according to any one of Claims 17 to 24, **characterized in that** 4-tert-butyl-4'-methoxydibenzoylmethane is present in the composition in a content ranging from 0.2% to 15% by weight relative to the total weight of the composition.

26. Use according to Claim 25, **characterized in that** the said content ranges from 0.5% to 10% by weight relative to the total weight of the composition.

27. Process for improving the stability to UV radiation of cosmetic and/or dermatological compositions including, 4-tert-butyl-4'-methoxydibenzoylmethane and a 1,3,5-triazine derivative as defined in any one of Claims 1 to 7, **characterized in that** it consists in introducing into the said compositions an effective quantity of an alkyl β,β'-diphenylacrylate or alkyl α-cyano-β,β'-diphenylacrylate.

28. Process according to Claim 27, **characterized in that** the alkyl β,β'-diphenylacrylate or alkyl α-cyano-β,β'-diphenylacrylate is 2-ethylhexyl α-cyano-β,β'-diphenylacrylate.

29. Process according to Claim 27, **characterized in that** the alkyl β,β'-diphenylacrylate or alkyl α-cyano-β,β'-diphenylacrylate is ethyl α-cyano-β,β'-diphenylacrylate.

30. Process according to any one of Claims 27 to 29, **characterized in that** the 1,3,5-triazine derivative is 2,4,6-tris[p-(2'-ethylhexyl-1'-oxycarbonyl)anilino]-1,3,5-triazine.

31. Process of cosmetic treatment for protecting the skin and/or the hair against UV radiation, in particular solar radiation, **characterized in that** it consists in applying thereto an effective quantity of a cosmetic composition such as in any one of Claims 1 to 16.

## Patentansprüche (Patentansprüche für folgende(n) Vertragsstaat(en): ES, GB, FR, IT)

1. Kosmetische und/oder dermatologische Zusammensetzung, die in einem kosmetisch und/oder dermatologisch akzeptablen Träger i) 4-*tert*-Butyl-4'-methoxy-dibenzoylmethan, ii) mindestens ein 1,3,5-Triazinderivat der folgenden Formel (I): 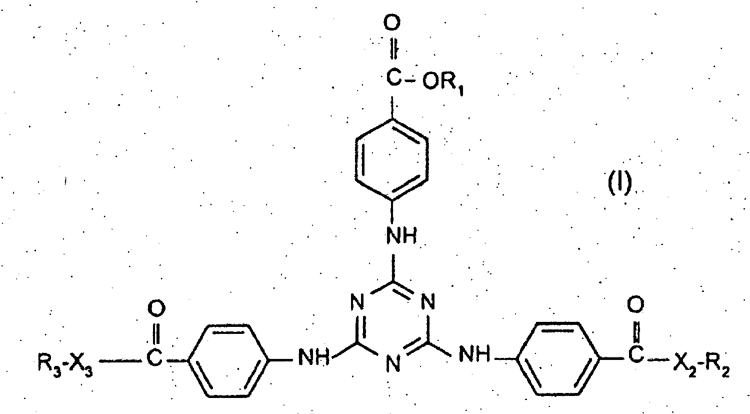 worin
- X₂ und X₃, die identisch oder voneinander verschieden sind, Sauerstoff oder die Gruppe -NH- bedeuten; und
- R₁, R₂ und R₃, die identisch oder voneinander verschieden sind, ausgewählt sind unter: Wasserstoff; einem Alkalimetall; einer Ammoniumgruppe, die gegebenenfalls mit einer oder mehreren Alkylgruppen oder Hydroxyalkylgruppen substituiert ist; einer linearen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer polyethoxylierten Gruppe, die 1 bis 6 Ethylenoxideinheiten aufweist und deren endständige OH-Gruppe methyliert ist, und einer Gruppe der folgenden Formel (II), (III) oder (IV): 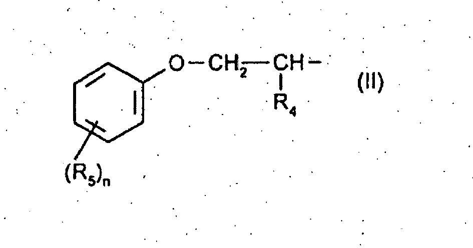 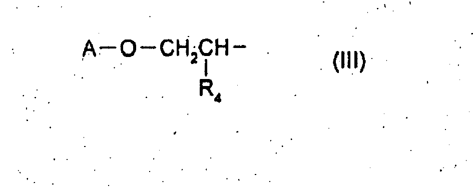 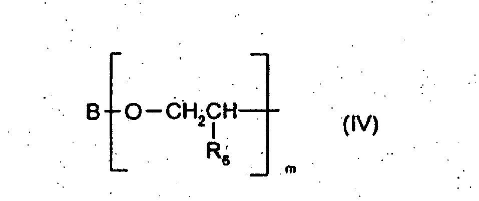
worin bedeuten:
- R₄ Wasserstoff oder Methyl;
- R₅ C₁₋₉-Alkyl;
- n Null oder eine ganze Zahl von 1 bis 3;
- m eine ganze Zahl von 1 bis 10;
- A C₄₋₈-Alkyl oder C₅₋₈-Cycloalkyl;
und worin
- B ausgewählt ist unter: einer linearen oder verzweigten C₁₋₈-Alkylgruppe; einer C₅₋₈-Cycloalkylgruppe und einer Arylgruppe, die gegebenenfalls mit einer oder mehreren C₁₋₄-Alkylgruppen substituiert ist;
und
- R₆ Wasserstoff oder Methyl bedeutet;
und
iii) mindestens ein Alkyl-β,β'-diphenylacrylat oder Alkyl-α-cyano-β,β'-diphenylacrylat der folgenden Formel (V) enthält: 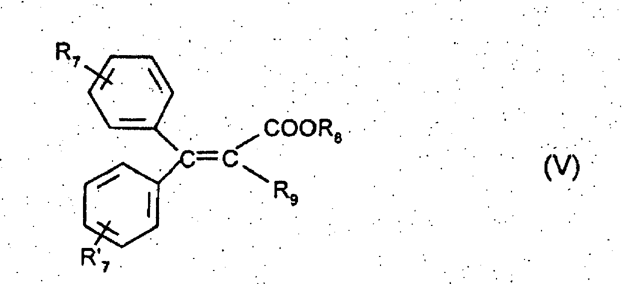
worin
- R₇ und R'₇, die identisch oder voneinander verschieden sind, in m-Stellung oder in p-Stellung vorliegen und ausgewählt sind unter: Wasserstoff, einer geradkettigen oder verzweigten C₁₋₈-Alkoxygruppe und einer geradkettigen oder verzweigten C₁₋₄-Allylgruppe;
- R₈ eine geradkettige oder verzweigte C₁₋₁₂-Alkylgruppe bedeutet;
und
- R₉ Wasserstoff oder eine Gruppe -CN darstellt;
wobei diese Zusammensetzung kein 2-Ethylhexyl-p-methoxy-cinnamat enthält, und
mit der Maßgabe, dass die Zusammensetzung von der folgenden Formulierung verschieden ist, bei der die Mengenanteile der verschiedenen Bestandteile in Gewichtsprozent, bezogen auf das Gesamtgewicht der Zusammensetzung, angegeben sind:
| Bestandteile | Mengen |
|---|---|
| Komponente I, UV-Filter | |
| 4-Methylbenzylidene Camphor (Eusolex 6300) UV-B-Filter | 3,0 |
| 2,4,6-Tris[p-(2'-ethylhexyl-1'-oxycarbonyl)anilino]- 1,3,5-triazin (Uvinul T 150) UV-B-Filter | - 1,5 |
| 2-Ethylhexyl Salicylate (Neo Heliopan OS) UV-B-Filter | 4,0 |
| 2-Ethylhexyl-α-cyano-β,β'-diphenylacrylat (Neo Heliopan 303) UV-B-Filter | 4,0 |
| 4-tert-Butyl-4'-methoxydibenzoylmethan (Parsol 1789) UV-A-Filter | 2,0 |
| Komponente II, Fettkomponenten | |
|---|---|
| C₁₂-C₁₅ Alkyl Benzoate (Finsolv TN) | 12,0 |
| Komponente III | |
|---|---|
| Parfümöl | 0,4 |
| Konservierungsmittel | qs |
| Komponente IV | |
|---|---|
| Carbomer | 0,8 |
| Glycerin | 3,0 |
| EDTA | 0,1 |
| Wasser ad | 100,0 |
| Komponente V | |
|---|---|
| Wasser | 20,0 |
| Novantisolsäure (wasserlöslicher UV-B-Filter) | 2,0 |
| Neutralisationsmittel (insbesondere NaOH, Triethanolamin) | qs |

2. Zusammensetzung nach Anspruch 1, **dadurch gekennzeichnet, dass** das 1,3,5-Triazinderivat unter den 1,3,5-Triazinderivaten ausgewählt ist, die alle nachstehenden Eigenschaften aufweisen:
- X₂ und X₃ sind identisch und bedeuten Sauerstoff;
- R₁ ist ausgewählt unter: einer C₅₋₁₂-Cycloalkylgruppe, die gegebenenfalls mit einer oder mehreren C₁₋₄-Alkylgruppen substituiert ist, und einer Gruppe der Formel (II), (III) oder (IV), worin bedeuten:
- B C₁₋₄-Alkyl; und
- R₆ Methyl;
- R₂ und R₃, die identisch oder voneinander verschieden sind, sind ausgewählt unter: Wasserstoff; einem Alkalimetall; einer Ammoniumgruppe, die gegebenenfalls mit einer oder mehreren Alkylgruppen oder Hydroxyalkylgruppen substituiert ist; einer linearen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; und einer Gruppe der Formel (II), (III) oder (IV), worin bedeuten:
- B C₁₋₄-Alkyl
und
- R₆ Methyl.

3. Zusammensetzung nach Anspruch 1, **dadurch gekennzeichnet, dass** das 1,3,5-Triazinderivat unter den 1,3,5-Triazinderivaten ausgewählt ist, die sämtliche der folgenden Eigenschaften aufweisen:
- X₂ und X₃ sind identisch und bedeuten die Gruppe -NH-;
- R₃ ist ausgewählt unter: einer linearen oder verzweigten C₁₋₁₈-Alkylgruppe und einer C₅₋₁₂-Cycloalkylgruppe, die gegebenenfalls mit einer oder mehreren C₁₋₄-Alkylgruppen substituiert ist;
- R₁ ist ausgewählt unter: Wasserstoff; einem Alkalimetall; einer Ammoniumgruppe; einer Gruppe der Formel (IV); einer linearen oder verzweigten C₁₋₁₈-Alkylgruppe und einer C₅₋₁₂-Cycloalkylgruppe, die gegebenenfalls mit einer oder mehreren C₁₋₄-Alkylgruppen substituiert ist;
- R₂ ist ausgewählt unter: einer linearen oder verzweigten C₁₋₁₈-Alkylgruppe und einer C₅₋₁₂-Cycloalkylgruppe, die gegebenenfalls mit einer oder mehreren C₁₋₄-Alkylgruppen substituiert ist.

4. Zusammensetzung nach Anspruch 1, **dadurch gekennzeichnet, dass** das 1,3,5-Triazinderivat unter den 1,3,5-Triazinderivaten ausgewählt ist, die alle nachstehenden Eigenschaften aufweisen:
- X₂ bedeutet Sauerstoff;
- X₃ bedeutet die Gruppe -NH-;
- R₃ ist ausgewählt unter: einer linearen oder verzweigten C₁₋₁₈-Alkylgruppe und einer C₅₋₁₂-Cycloalkylgruppe, die gegebenenfalls mit einer oder mehreren C₁₋₄-Alkylgruppen substituiert ist;
- R₁ ist ausgewählt unter: Wasserstoff; einem Alkalimetall; einer Ammoniumgruppe; einer Gruppe der Formel (IV); einer linearen oder verzweigten C₁₋₁₈-Alkylgruppe und einer C₅₋₁₂-Cycloalkylgruppe, die gegebenenfalls mit einer oder mehreren C₁₋₄-Alkylgruppen substituiert ist;
- R₂ ist ausgewählt unter: Wasserstoff; einem Alkalimetall; einer Ammoniumgruppe; einer Gruppe der Formel (IV); einer linearen oder verzweigten C₁₋₁₈-Alkylgruppe und einer C₅₋₁₂-Cycloalkylgruppe, die gegebenenfalls mit einer oder mehreren C₁₋₄-Alkylgruppen substituiert ist.

5. Zusammensetzung nach Anspruch 4, **dadurch gekennzeichnet, dass** es sich bei dem 1,3,5-Triazinderivat um das 1,3,5-Triazinderivat der folgenden Formel handelt: 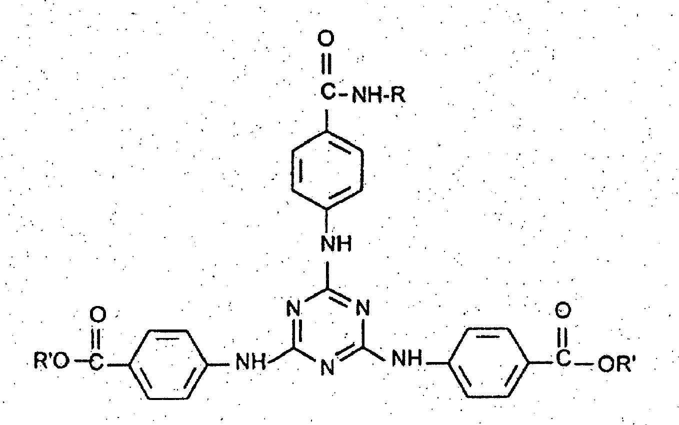 worin R' 2-Ethylhexyl und R *tert*-Butyl bedeuten.

6. Zusammensetzung nach Anspruch 1, **dadurch gekennzeichnet, dass** das 1,3,5-Triazinderivat unter den 1,3,5-Triazinderivaten ausgewählt ist, die alle nachstehenden Eigenschaften aufweisen:
- X₂ und X₃ sind identisch und bedeuten Sauerstoff;
- R₁, R₂ und R₃ sind identisch und bedeuten eine C₆₋₁₂-Alkylgruppe oder eine polyethoxylierte Gruppe, die 1 bis 6 Ethylenoxideinheiten aufweist und deren endständige OH-Gruppe methyliert ist.

7. Zusammensetzung nach Anspruch 6, **dadurch gekennzeichnet, dass** es sich bei dem 1,3,5-Triazinderivat um das 1,3,5-Triazinderivat der folgenden Formel handelt: 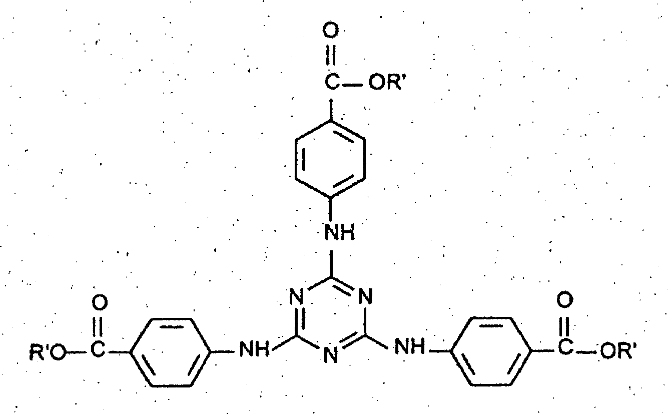 worin R' 2-Ethylhexyl bedeutet.

8. Zusammensetzung nach einem der Ansprüche 1 bis 7, **dadurch gekennzeichnet, dass** das 1,3,5-Triazinderivat in der Zusammensetzung in einem Mengenanteil von 0,5 bis 20 Gew.-%, bezogen auf das Gesamtgewicht der Zusammensetzung, vorliegt.

9. Zusammensetzung nach Anspruch 8, **dadurch gekennzeichnet, dass** dieser Mengenanteil im Bereich von 1 bis 10 Gew.-%, bezogen auf das Gesamtgewicht der Zusammensetzung, liegt.

10. Zusammensetzung nach einem der Ansprüche 1 bis 9, **dadurch gekennzeichnet, dass** es sich bei dem Alkyl-β,β'-diphenylacrylat oder Alkyl-α-cyano-β,β'-diphenylacrylat um 2-Ethylhexyl-α-cyano-β,β'-diphenylacrylat handelt.

11. Zusammensetzung nach einem der Ansprüche 1 bis 9, **dadurch gekennzeichnet, dass** es sich bei dem Alkyl-β,β'-diphenylacrylat oder Alkyl-α-cyano-β,β'-diphenylacrylat um Ethyl-α-cyano-β,β'-diphenylacrylat handelt.

12. Zusammensetzung nach einem der Ansprüche 1 bis 11, **dadurch gekennzeichnet, dass** das Alkyl-β,β'-diphenylacrylat oder Alkyl-α-cyano-β,β'-diphenylacrylat in der Zusammensetzung in einem Mengenanteil von mindestens 0,5 Gew.-%, bezogen auf das Gesamtgewicht der Zusammensetzung, vorliegt.

13. Zusammensetzung nach Anspruch 12, **dadurch gekennzeichnet, dass** dieser Mengenanteil im Bereich von 0,5 bis 20 Gew.-%, bezogen auf das Gesamtgewicht der Zusammensetzung, liegt.

14. Zusammensetzung nach einem der Ansprüche 1 bis 13, **dadurch gekennzeichnet, dass** das 4-*tert*-Butyl-4'-methoxy-dibenzoylmethan in den Zusammensetzungen in einem Mengenanteil von 0,2 bis 15 Gew.-%, bezogen auf das Gesamtgewicht der Zusammensetzung, vorliegt.

15. Zusammensetzung nach Anspruch 14, **dadurch gekennzeichnet, dass** dieser Mengenanteil im Bereich von 0,5 bis 10 Gew.-%, bezogen auf das Gesamtgewicht der Zusammensetzung, liegt.

16. Zusammensetzung nach einem der Ansprüche 1 bis 15, **dadurch gekennzeichnet, dass** sie in Form einer Öl-in-Wasser-Emulsion vorlegt.

17. Verwendung eines Alkyl-β,β'-diphenylacrylats oder Alkyl-α-cyano-β,β'-diphenylacrylats in oder zur Herstellung von kosmetischen und/oder dermatologischen Zusammensetzungen, die 4-*tert*-Butyl-4'-methoxy-dibenzoylmethan in Kombination mit mindestens einem 1,3,5-Triazinderivat nach einem der Ansprüche 1 bis 7 enthalten, um in diesen Zusammensetzungen die Stabilität dieses 1,3,5-Triazinderivats gegenüber UV-Strahlung zu verbessern.

18. Verwendung nach Anspruch 17, **dadurch gekennzeichnet, dass** es sich bei dem Alkyl-β,β'-diphenylacrylat oder Alkyl-α-cyano-β,β'-diphenylacrylat um 2-Ethylhexyl-α-cyano-β,β'-diphenylacrylat handelt.

19. Verwendung nach Anspruch 17, **dadurch gekennzeichnet, dass** es sich bei dem Alkyl-β,β'-diphenylacrylat oder Alkyl-α-cyano-β,β'-diphenylacrylat um Ethyl-α-cyano-β,β'-diphenylacrylat handelt.

20. Verwendung nach einem der Ansprüche 17 bis 19, **dadurch gekennzeichnet, dass** es sich bei dem 1,3,5-Triazinderivat um 2,4,6-Tris[p-(2'-ethylhexyl-1'-oxycarbonyl)anilino]-1,3,5-triazin handelt.

21. Verwerdung nach einem der Ansprüche 17 bis 20, **dadurch gekennzeichnet, dass** das 1,3,5-Triazinderivat in der Zusammensetzung in einem Mengenanteil von 0,5 bis 20 Gew.-%, bezogen auf das Gesamtgewicht der Zusammensetzung, vorliegt.

22. Verwendung nach Anspruch 21, **dadurch gekennzeichnet, dass** dieser Mengenanteil im Bereich von 1 bis 10 Gew.-%, bezogen auf das Gesamtgewicht der Zusammensetzung, liegt.

23. Verwendung nach einem der Ansprüche 17 bis 22, **dadurch gekennzeichnet, dass** das Alkyl-β,β'-diphenylacrylat oder Alkyl-α-cyano-β,β'-diphenylacrylat in der Zusammensetzung in einem Mengenanteil von mindestens 0,5 Gew.-%, bezogen auf das Gesamtgewicht der Zusammensetzung, vorliegt.

24. Verwendung nach Anspruch 23, **dadurch gekennzeichnet, dass** dieser Mengenanteil im Bereich von 0,5 bis 20 Gew.-%, bezogen auf das Gesamtgewicht der Zusammensetzung, liegt.

25. Verwendung nach einem der Ansprüche 17 bis 24, **dadurch gekennzeichnet, dass** das 4-*tert*-Butyl-4'-methoxy-dibenzoylmethan in der Zusammensetzung in einem Mengenanteil von 0,2 bis 15 Gew.-%, bezogen auf das Gesamtgewicht der Zusammensetzung, vorliegt.

26. Verwerdung nach Anspruch 25, **dadurch gekennzeichnet, dass** dieser Mengenanteil im Bereich von 0,5 bis 10 Gew.-%, bezogen auf das Gesamtgewicht der Zusammensetzung, liegt.

27. Verfahren zur Verbesserung der Stabilität kosmetischer und/oder dermatologischer Zusammensetzungen, die 4-*tert-*Butyl-4'-methoxy-dibenzoylmethan und ein 1,3,5-Triazinderivat nach einem der Ansprüche 1 bis 7 enthalten, gegenüber UV-Strahlung, **dadurch gekennzeichnet, dass** es darin besteht, in diese Zusammensetzungen eine wirksame Menge eines Alkyl-β,β'-diphenylaclylats oder Alkyl-α-cyano-β,β'-diphenylacrylats einzubringen.

28. Verfahren nach Anspruch 27, **dadurch gekennzeichnet, dass** es sich bei dem Alkyl-β,β'-diphenylacrylat oder Alkyl-α-cyano-β,β'-diphenylacrylat um 2-Ethylhexyl-α-cyano-β,β'-diphenylacrylat handelt.

29. Verfahren nach Anspruch 27, **dadurch gekennzeichnet, dass** es sich bei dem Alkyl-β,β'-diphenylacrylat oder Alkyl-α-cyano-β,β'-diphenylacrylat um Ethyl-α-cyano-β,β'-diphenylacrylat handelt.

30. Verfahren nach einem der Ansprüche 27 bis 29, **dadurch gekennzeichnet, dass** es sich bei dem 1,3,5-Triazinderivat um 2,4,6-Tris[p-(2'-ethylhexyl-1'-oxycarbonyl)anilino]-1,3,5-triazin handelt.

31. Verfahren zur kosmetischen Behandlung, um die Haut und/ oder die Haare gegen UV-Strahlung, insbesondere gegen Sonnenlicht, zu schützen, **dadurch gekennzeichnet, dass** es darin besteht, auf die Haut und/oder die Haare eine wirksame Menge einer kosmetischen Zusammensetzung nach einem der Ansprüche 1 bis 16 aufzutragen.

## Patentansprüche (Patentansprüche für folgende(n) Vertragsstaat(en): DE)

1. Kosmetische und/oder dermatologische Zusammensetzung, die in einem kosmetisch und/oder dermatologisch akzeptablen Träger i) 4-*t*ert-Butyl-4'-methoxy-dibenzoylmethan, ii) mindestens ein 1,3,5-Triazinderivat der folgenden Formel (I): 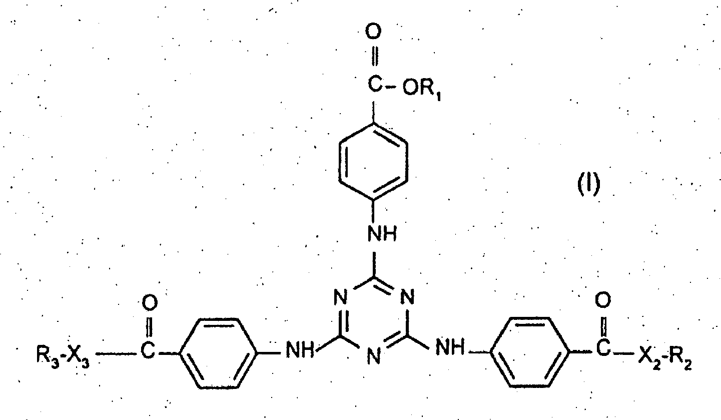 worin
- X₂ und X₃, die identisch oder voneinander verschieden sind, Sauerstoff oder die Gruppe -NH- bedeuten;
und
- R₁, R₂ und R₃, die identisch oder voneinander verschieden sind, ausgewählt sind unter: Wasserstoff; einem Alkalimetall; einer Ammoniumgruppe, die gegebenenfalls mit einer oder mehreren Alkylgruppen oder Hydroxyalkylgruppen substituiert ist; einer linearen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer polyethoxylierten Gruppe, die 1 bis 6 Ethylenoxideinheiten aufweist und deren endständige OH-Gruppe methyliert ist, und einer Gruppe der folgenden Formel (II), (III) oder (IV): 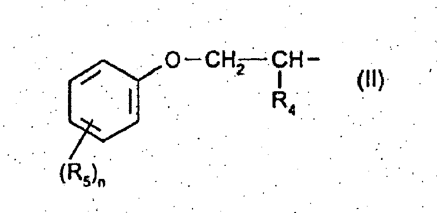 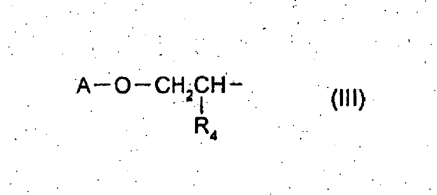 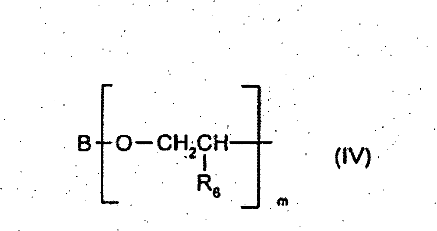
worin bedeuten:
- R₄ Wasserstoff oder Methyl;
- R₅ C₁₋₉-Alkyl;
- n Null oder eine ganze Zahl von 1 bis 3;
- m eine ganze Zahl von 1 bis 10;
- A C₄₋₈-Alkyl oder C₅₋₈-Cycloalkyl;
und worin
- B ausgewählt ist unter: einer linearen oder verzweigten C₁₋₈-Alkylgruppe; einer C₅₋₈-Cycloalkylgruppe und einer Arylgruppe, die gegebenenfalls mit einer oder mehreren C₁₋₄-Alkylgruppen substituiert ist;
und
- R₆ Wasserstoff oder Methyl bedeutet; und
iii) mindestens ein Alkyl-β,β'-diphenylacrylat oder Alkyl-α-cyano-β,β'-diphenylacrylat der folgenden Formel (V) enthält: 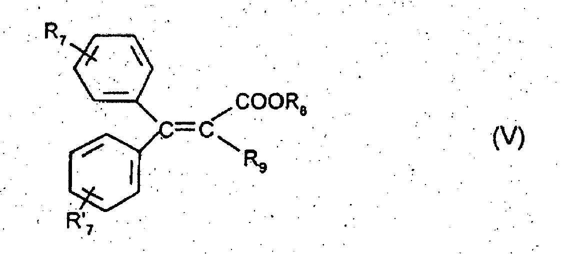
worin
- R₇ und R'₇, die identisch oder voneinander verschieden sind, in m-Stellung oder in p-Stellung vorliegen und ausgewählt sind unter: Wasserstoff, einer geradkettigen oder verzweigten C₁₋₈-Alkoxgruppe und einer geradkettigen oder verzweigten C₁₋₄-Alkylgruppe;
- R₈ eine gerädkettige oder verzweigte C₁₋₁₂-Alkylgruppe bedeutet;
und
- R₉ Wasserstoff oder eine Gruppe -CN darstellt;
wobei diese Zusammensetzung kein 2-Ethylhexyl-p-methoxycinnamat enthält, und
mit der Maßgabe, dass die Zusammensetzung von den folgenden Formulierungen verschieden ist, bei denen die Mengenanteile der verschiedenen Bestandteil in Gewichtsprozent, bezogen auf das Gesamtgewicht der Zusammensetzung, angegeben sind:
| Zusammensetzung 1 | |
|---|---|
| Bestandteile | Mengen |
| Komponente I, UV-Filter | |
| 4-Methylbenzylidene Camphor (Eusolex 6300) UV-B-Filter | 3,0 |
| 2,4,6-Tris[p-(2'-ethylhexyl-1'-oxycarbonyl)anilino]-1,3,5-triazin (Uvinul T 150) UV-B-Filter | 1,5 |
| 2-Ethylhexyl Salicylate (Neo Heliopan OS) UV-B-Filter | 4,0 |
| 2-Ethylhexyl-α-cyano-β,β'-diphenylacrylat (Neo Heliopan 303) UV-B-Filter | 4,0 |
| 4-tert-Butyl-4'-methoxydibenzoylmethan (Parsol 1789) UV-A-Filter | 2,0 |
| Komponente II, Fettkomponenten | |
|---|---|
| C₁₂-C₁₅ Alkyl Benzoate (Finsolv TN) | 112,0 |
| Komponente III | |
|---|---|
| Parfümöl | 0,4 |
| Konservierungsmittel | qs |
| Komponente IV | |
|---|---|
| Carbomer | 0,8 |
| Glycerin | 3,0 |
| EDTA | 0,1 |
| Wasser ad | 100,0 |
| Komponente V | |
|---|---|
| Wasser | 20,0 |
| Novantisolsäure (wasserlöslicher UV-B-Filter) | 2,0 |
| Neutralisationsmittel (insbesondere NaOH, Tri-ethanolamin) | qs |
| Zusammensetzung 2 | |
|---|---|
| Bestandteile | Mengen |
| Komponente I, UV-Filter | |
| 4-Methylbenzylidene Camphor (Eusolex 6.300) UV-B-Filter | 4,0 |
| 2,4,6-Tris[p-(2'-ethylhexyl-1'-oxycarbonyl)anilino]-1,3,5-triazin (Uvinul T 150) UV-B-Filter | 2,0 |
| 2-Ethylhexyl Salicylate (Neo Heliopan OS) UV-B-Filter | 3,0 |
| 2-Ethylhexyl-α-cyano-β,β'-diphehylacrylat (Neo Heliopan 303) UV-B-Filter | 3,0 |
| 4-tert-Butyl-4'-methoxydibenzoylmethan (Parsol 1789) UV-A-Filter | 3,0 |
| Komponente II, Fettkomponenten | |
|---|---|
| C₁₂-C₁₅ Alkyl Benzoate (Finsolv TN) | 12,0 |
| Caprylic/Capric Triglycerides (Miglyol 812) | 2,0 |
| Octyl Stearate (Cetiol 868) | 3,0 |
| Komponente III. | |
|---|---|
| Parfümöl | 0,4 |
| Konservierungsmittel | qs |
| Komponente IV | |
|---|---|
| Carbomer | 0,4 |
| Glycerin | 3,0 |
| EDTA | 0,1 |
| Wasser ad | 100,0 |
| Komponente V | |
|---|---|
| Neutralisationsmittel (insbesondere NaOH, Tri-ethanolamin) | qs |

2. Zusammensetzung nach Anspruch 1, **dadurch gekennzeichnet, dass** das 1,3,5-Triazinderivat unter den 1,3,5-Triazinderivaten ausgewählt ist, die alle nachstehenden Eigenschaften aufweise: X₂ und X₃ sind identisch und bedeuten Sauerstoff;
- R₁ ist ausgewählt unter: einer C₅₋₁₂-Cycloalkylgruppe, die gegebenenfalls mit einer oder mehreren C₁₋₄-Alkylgruppen substituiert ist, und einer Gruppe der Formel (II), (III) oder (IV), worin bedeuten:
- BC₁₋₄-Alkyl; und
- R₆ Methyl;
- R₂ und R₃, die identisch oder voneinander verschieden sind, sind ausgewählt unter: Wasserstoff; einem Alkalimetall; einer Ammoniumgruppe, die gegebenenfalls mit einer oder mehreren Alkylgruppen oder Hydroxyalkylgruppen substituiert ist; einer linearen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; und einer Gruppe der Formel (II), (III) oder (IV), worin bedeuten:
- B C₁₋₄-Alkyl
und
- R₆ Methyl.

3. Zusammensetzung nach Anspruch 1, **dadurch gekennzeichnet, dass** das 1,3,5-Triazinderivat unter den 1,3,5'-Triazinderivaten ausgewählt ist, die sämtliche der folgenden Eigenschaften aufweisen:
- X₂ und X₃ sind identisch und bedeuten die Gruppe -NH-;
- R₃ ist ausgewählt unter: einer linearen oder verzweigten C₁₋₁₈-Alkylgruppe und einer C₅₋₁₂-Cycloalkylgruppe, die gegebenenfalls mit einer oder mehreren C₁₋₄-Alkylgruppen substituiert ist;
- R₁ ist ausgewählt unter: Wasserstoff; einem Alkalimetall; einer Ammoniumgruppe; einer Gruppe der Formel (IV); einer linearen oder verzweigten C₁₋₁₈-Alkylgruppe und einer C₅₋₁₂-Cycloalkylgruppe, die gegebenenfalls mit einer oder mehreren C₁₋₄-Alkylgruppen substituiert ist;
- R₂ ist ausgewählt unter: einer linearen oder verzweigten C₁₋₁₈-Alkylgruppe und einer C₅₋₁₂-Cycloalkylgruppe, die gegebenenfalls mit einer oder mehreren C₁₋₄-Alkylgruppen substituiert ist.

4. Zusammensetzung nach Anspruch 1, **dadurch gekennzeichnet, dass** das 1,3,5-Triazinderivat unter den 1,3,5-Triazinderivaten ausgewählt ist, die alle nachstehenden Eigenschaften aufweisen:
- X₂ bedeutet Sauerstoff;
- X₃ bedeutet die Gruppe-NH-;
- R₃ ist ausgewählt unter: einer linearen Oder verzweigten C₁₋₁₈-Alkylgruppe und einer C₅₋₁₂-Cycloalkylgruppe, die gegebenenfalls mit einher oder mehreren C₁₋₄-Alkylgruppen substituiert ist;
- R₁ ist ausgewählt unter: Wasserstoff; einem Alkalimetall; einer Ammoniumgruppe; einer Gruppe der Formel (IV); einer linearen oder verzweigten C₁₋₁₈-Alkylgruppe und einer C₅₋₁₂-Cycloalkylgruppe, die gegebenenfalls mit einer oder mehreren C₁₋₄-Alkylgruppen substituiert ist;
- R₂ ist ausgewählt unter: Wasserstoff; einem Alkalimetall; einer Ammoniumgruppe; einer Gruppe der Formel (IV); einer linearen oder verzweigten C₁₋₁₈-Alkylgruppe und einer C₅₋₁₂-Cycloalkylgruppe, die gegebenenfalls mit einer öder mehreren C₁₋₄-Alkylgruppen substituiert ist.

5. Zusammensetzung nach Anspruch 4, **dadurch gekennzeichnet, dass** es sich bei dem 1,3,5-Triazinderivat um das 1,3,5-Triazinderivat der folgenden Formel handelt: 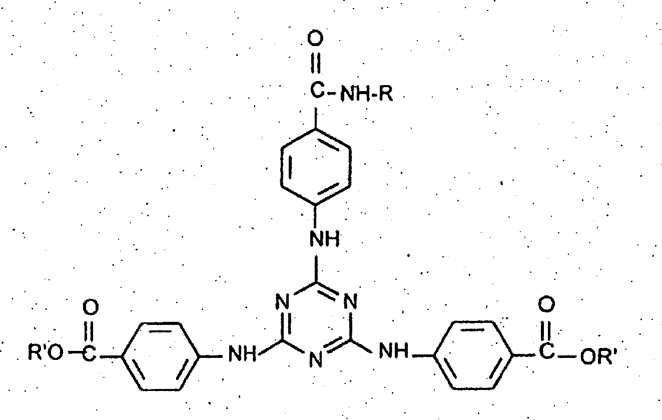 worin R' 2-Ethylhexyl und R *tert*-Butyl bedeuten.

6. Zusammensetzung nach Anspruch 1, **dadurch gekennzeichnet, dass** das 1,3,5-Triazinderivat unter den 1,3,5-Triazinderivaten ausgewählt ist, die all nachstehenden Eigenschaften aufweisen:
- X₂ und X₃ sind identisch und bedeuten Sauerstoff;
- R₁, R₂ und R₃ sind identisch und bedeuten eine C₆₋₁₂-Alkylgruppe oder eine polyethoxylierte Gruppe, die 1 bis 6 Ethylenoxideinheiten aufweist und deren endständige OH-Gruppe methyliert ist.

7. Zusammensetzung nach Anspruch 6, **dadurch gekennzeichnet, dass** es sich bei dem 1,3,5-Triazinderivat um das 1,3,5-Triazinderivat der folgenden Formel handelt: 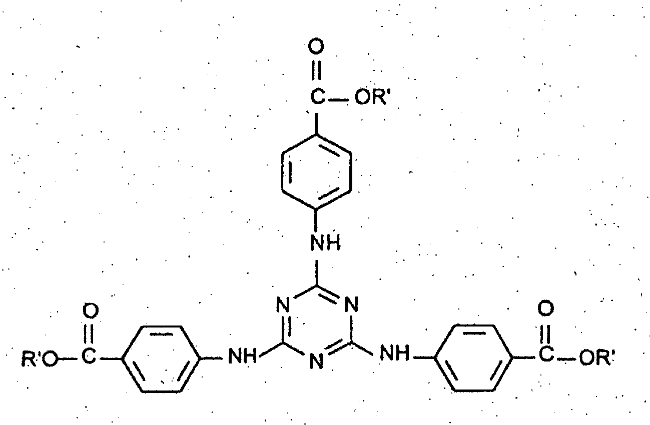 worin R' 2-Ethylhexyl bedeutet.

8. Zusammensetzung nach einem der Ansprüche 1 bis 7, **dadurch gekennzeichnet, dass** das 1,3,5-Triazinderivat in der Zusammensetzung in einem Mengenanteil von 0,5 bis 20 Gew.-%, bezogen auf das Gesamtgewicht der Zusammensetzung, vorliegt.

9. Zusammensetzung nach Anspruch 8, **dadurch gekennzeichnet, dass** dieser Mengenanteil im Bereich von 1 bis 10 Gew.-%, bezogen auf das Gesamtgewicht der Zusammensetzung, liegt.

10. Zusammensetzung nach einem der Ansprüche 1 bis 9, **dadurch gekennzeichnet, dass** es sich bei dem Alkyl-β,β'-diphenylacrylat oder Alkyl-α-cyano-β,β'-diphenylacrylat um 2-Ethylhexyl-α-cyano-β,β'-diphenylacrylat handelt.

11. Zusammensetzung nach einem der Ansprüche 1 bis 9, **dadurch gekennzeichet, dass** es sich bei dem Alkyl-β,β.'-diphenylacrylat oder Alkyl-α-cyano-β,β'-diphenylacrylat um Ethyl-α-cyano-β,β'-diphenylacrylat handelt.

12. Zusammensetzung nach einem der Ansprüche 1 bis 11, **dadurch gekennzeichnet, dass** das Alkyl-β,β'-diphenylacrylat oder Alkyl-α-cyano-β,β'-diphenylacrylat in der Zusammensetzung in einem Mengenanteil von mindestens 0,5 Gew.-%, bezogen auf das Gesamtgewicht der Zusammensetzung, vorliegt.

13. Zusammensetzung nach Anspruch 12, **dadurch gekennzeichnet, dass** dieser Mengenanteil im Bereich von 0,5 bis 20 Gew.-%, bezogen auf das Gesamtgewicht der Zusammensetzung, liegt.

14. Zusammensetzung nach einem der Ansprüche 1 bis 13, **dadurch gekennzeichnet, dass** das 4-*tert*-Butyl-4'-methoxy-dibenzoylmethan in den Zusammensetzungen in einem Mengenanteil von 0,2 bis 15 Gew.-%, bezogen auf das Gesamtgewicht der Zusammensetzung, vorliegt.

15. Zusammensetzung nach Anspruch 14, **dadurch gekennzeichnet, dass** dieser Mengenanteil im Bereich von 0,5 bis 10 Gew.-%, bezogen auf das Gesamtgewicht der Zusammensetzung, liegt.

16. Zusammensetzung nach einem der Ansprüche 1 bis 15, **dadurch gekennzeichnet, dass** sie in Form einer Öl-in-Wasser-Emulsion vorliegt.

17. Verwendung eines Alkyl-β,β'-diphenylacrylats oder Alkyl-α-cyano-β,β'-diphenylacrylats in oder zur Herstellung von kosmetischen und/oder dermatologischen Zusammensetzungen, die 4-*tert*-Butyl-4'-methoxy-dibenzoylmethan in Kombination mit mindestens einem 1,3,5-Triazinderivat nach einem der ansprüche 1 bis 7 enthalten, um in diesen Zusammensetzungen die Stabilität dieses 1,3,5-Triazinderivats gegenüber UV-Strahlung zu verbessern.

18. Verwendung nach Anspruch 17, **dadurch gekennzeichnet, dass** es sich bei dem Alkyl-β,β'-diphenylacrylat oder Alkyl-α-cyano-β, β'-dipheriylacrylat um 2-Ethylhexyl-α-cyarlo-β,β'-diphenylacrylat handelt.

19. Verwendung nach Anspruch 17, **dadurch gekennzeichnet, dass** es sich bei dem Alkyl-β,β'-diplienylacrylat oder Alkyl-α-cyαno-β,β'-diphenyläciylat um Ethyl-α-cyano=β,β'-diphenylacrylat handelt.

20. Verwendung nach einem der Ansprüche 17 bis 19, **dadurch gekennzeichnet, dass** es sich bei dem 1,3,5-Triazinderivat um 2,4,6-Tris[p-(2'-ethylhexyl-1'-oxycarbonyl)anilino]-1,3,5-triazin handelt.

21. Verwendung nach einem der Ansprüche 17 bis 20, **dadurch gekennzeichnet, dass** das 1,3,5-Triazinderivat in der Zusammensetzung in einem Mengenanteil von 0,5 bis 20 Gew.-%, bezogen auf das Gesamtgewicht der Zusammensetzung, vorliegt.

22. verwendung nach Anspruch 21, **dadurch gekennzeichnet, dass** dieser Mengenanteil im Bereich von 1 bis 10 Gew.-%, bezogen auf das Gesamtgewicht der Zusammensetzung, liegt.

23. Verwendung nach einem der Ansprüche 17 bis 22, **dadurch gekennzeichnet, dass** das Alkyl-β,β'-diphenylacrylat oder Alkyl-α-cyano-β,β'-diphenylacrylat in der Zusammensetzung in einem Mengenanteil von mindestens 0,5 Gew.-%, bezogen auf das Gesamtgewicht der Zusammensetzung, vorlegt.

24. Verwerdung nach Anspruch 23, **dadurch gekennzeichnet, dass** dieser Mengenanteil im Bereich von 0,5 bis 20 Gew.-%, bezogen auf das Gesamtgewicht der Zusammensetzung, liegt.

25. Verwendung nach einem der Ansprüche 17 bis 24, **dadurch gekennzeichnet, dass** das 4-*tert*-Butyl-4'-methoxy-dibenzoylmethan in der Zusammensetzung in einem Mengenanteil von 0,2 bis 15 Gew.-%, bezogen auf das Gesamtgewicht der Zusammensetzung, vorliegt.

26. Verwerdung nach Anspruch 25, **dadurch gekennzeichnet, dass** dieser Mengenanteil im Bereich von 0,5 bis 10 Gew.-%, bezogen auf das Gesamtgewicht der Zusammensetzung, liegt.

27. Verfahren zur Verbesserung der Stabilität kosmetischer und/oder dermatologischer Zusammensetzungen, die 4-*tert-*Butyl-4'-methoxy-dibenzoylmethan und ein 1,3,5-Triazinderivat nach einem der Ansprüche 1 bis 7 enthalten, gegenüber UV-Strahlung, **dadurch gekennzeichnet, dass** es darin besteht, in diese Zusammensetzungen eine wirksame Menge eines Alkyl-β,β'-diphenylacrylats oder Alkyl-α-cyano-β,β'-diphenylacrylats einzubringen.

28. Verfahren nach Anspruch 27, **dadurch gekennzeichnet, dass** es sich bei dem Alkyl-β,β'-diphenylacrylat oder Alkyl-α-cyano-β,β'-diphenylacrylat um 2-Ethylhexyl-α-cyano-β,β'-diphenylacrylat handelt.

29. Verfahren nach Anspruch 27, **dadurch gekennzeichnet, dass** es sich bei dem Alkyl-β,β'-diphenylacrylat oder Alkyl-α-cyano-β,β'-diphenylacrylat um Ethyl-α-cyano-β,β'-diphenylacrylat handelt.

30. Verfahren nach einem der Ansprüche 27 bis 29, **dadurch gekennzeichnet, dass** es sich bei dem 1,3,5-Triazinderivat um 2,4,6-Tris[p-(2'-ethylhexyl-1'-oxycarbonyl)anilino]-1 ,3,5-triazin handelt.

31. Verfahren zur kosmetischen Behandlung, um die Haut und/ oder die Haare gegen UV-Strahlung, insbesondere gegen Sonnenlicht, zu schützen, **dadurch gekennzeichnet, dass** es darin besteht, auf die Haut und/oder die Haare eine wirksame Menge einer kosmetischen Zusammensetzung nach einem der Ansprüche 1 bis 16 auszutragen.
